# EUROPÄISCHE PATENTSCHRIFT

(11) **EP 0 389 425 B1**
(45) Veröffentlichungstag und Bekanntmachung des Hinweises auf die Patenterteilung: **26.04.1995**
(21) Anmeldenummer: 90810193.4
(22) Anmeldetag: 13.03.1990
(51) Int. Cl.: C07D 335/06, A61K 31/38, C07D 409/06

(54) **Neue Benzothiopyranylamine**
Benzotiopyranyl amines
Benzothiopyranylamines

(30) Priorität: 22.03.1989 US 326949
(43) Veröffentlichungstag der Anmeldung: 26.09.1990
(73) Patentinhaber: CIBA-GEIGY AG, 4002 Basel (CH)
(72) Erfinder: Hutchison, Alan J., Madison, Connecticut 06443 (US)

(56) Entgegenhaltungen:
- DE-A- 2 106 045
- US-A- 4 801 605

## Beschreibung

Die vorliegende Erfindung betrifft 3,4-Dihydro-2H-1-benzothiopyran-3-ylmethyl- und -ethylamine, die antipsychotisch wirksam sind, Verfahren zur Herstellung dieser Verbindungen, pharmazeutische Zusammensetzungen, die diese enthalten, und ein Verfahren zur Behandlung psychotischer Störungen mit diesen Verbindungen.

Die Erfindung betrifft insbesondere Benzothiopyranylmethyl- und -ethylamine der Formel 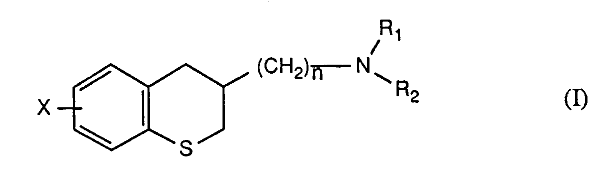
worin n 1 oder 2 ist, X Wasserstoff, Halogen, Niederalkyl, Hydroxy oder Niederalkoxy bedeutet, R₁ Niederalkyl ist, R₂ durch A substituiertes Niederalkyl ist, oder R₁ und R₂ zusammen Alkylen mit 4 bis 6 Kohlenstoffatomen darstellen, das durch A substituiert ist, und worin A Wasserstoff, Hydroxymethyl, α-Hydroxyarylmethyl, α-Hydroxydiarylmethyl, Niederalkoxymethyl, Arylniederalkoxymethyl, Niederalkanoyloxymethyl, Arylniederalkanoyloxymethyl, Aroyloxymethyl, Niederalkanoyl, Arylniederalkanoyl, Aroyl, Niederalkoxycarbonyl oder Arylniederalkoxycarbonyl bedeutet, und Salze dieser Verbindungen.

Die hierin verwendeten allgemeinen Definitionen haben in der vorliegenden Erfindung die folgende Bedeutung:
Der im Zusammenhang mit organischen Gruppen und Resten verwendete Ausdruck "nieder" bedeutet, dass die so bezeichneten Reste und Gruppen bis zu und einschliesslich 7, vorzugsweise bis zu und einschliesslich 4, und insbesondere 1 oder 2 Kohlenstoffatome enthalten.

Eine Niederalkylgruppe enthält 1 bis 7 Kohlenstoffatome, vorzugsweise 1 bis 4 Kohlenstoffatome, ist insbesondere geradkettig und stellt beispielsweise Methyl, Ethyl, Propyl oder Butyl dar.

Alkylen von 4 bis 6 Kohlenstoffatomen (für R₁ und R₂ zusammen) bedeutet vorzugsweise geradkettiges Butylen, Pentylen oder Hexylen und bildet zusammen mit dem Stickstoffatom Pyrrolidino, Piperidino beziehungsweise Perhydroazepino.

Eine Niederalkoxygruppe enthält vorzugsweise 1 bis 4 Kohlenstoffatome und ist beispielsweise Ethoxy, Propoxy, Isopropoxy oder insbesondere Methoxy.

Eine Niederalkanoylgruppe enthält 1 bis 7 Kohlenstoffatome, vorzugsweise 1 bis 4 Kohlenstoffatome, und ist beispielsweise Formyl, Acetyl, Propionyl, n-Butyryl, Isobutyryl oder n-Hexanoyl, vorzugsweise Acetyl oder Propionyl.

Aryl bedeutet einen carbocyclischen oder heterocyclischen aromatischen Rest, beispielsweise unsubstituiertes oder substituiertes Phenyl, Naphthyl, Furyl, Thienyl, Pyrrolyl, Thiazolyl oder Pyridyl. Aryl ist vorzugsweise Phenyl oder durch einen, zwei oder drei, vorzugsweise einen, Rest substituiertes Phenyl, wobei der Rest Niederalkyl, beispielsweise Methyl, Phenyl, Hydroxy, Niederalkoxy, beispielsweise Methoxy, Halogen, beispielsweise Chlor oder Fluor, oder Trifluoromethyl sein kann. Derart substituiertes Phenyl ist beispielsweise o-, m- oder p-Tolyl, 3,4-Xylyl, 2,6-Xylyl, p-Biphenylyl, p-Hydroxyphenyl, o- oder p-Methoxyphenyl, 3,4-Dimethoxyphenyl, o-, m- oder p-Chlorphenyl, 2,4- oder 3,4-Dichlorphenyl, 3-Chlor-4-methylphenyl, m- oder p-Fluorphenyl, 2,4-Difluorphenyl, 3-Chlor-4-fluorphenyl, 4-Chlor-3-fluorphenyl, m- oder p-Trifluormethylphenyl oder 4-Chlor-3-trifluormethylphenyl.

Aroyl ist einer der genannten carbocyclischen oder heterocyclischen aromatischen Reste, welcher mit Carbonyl verbunden ist, beispielsweise Naphthoyl, Furoyl, Thenoyl, Pyrrolylcarbonyl oder Pyridylcarbonyl, oder insbesondere Benzoyl oder substituiertes Benzoyl, worin die Substituenten die genannten Bedeutungen haben, beispielsweise o-, m- oder p-Toluoyl, p-Phenylbenzoyl, p-Hydroxybenzoyl, p-Anisoyl, p-Chlor- oder p-Fluorbenzoyl oder m-Trifluormethylbenzoyl.

Arylniederalkyl ist vorzugsweise Benzyl oder 2-Phenylethyl, wobei der Phenylring wie unter Aryl beschrieben substituiert sein kann.

Arylniederalkanoyl ist vorzugsweise Phenylacetyl, wobei der Phenylring wie unter Aryl beschrieben substituiert sein kann.

Halogen bedeutet vorzugsweise Chlor oder Fluor, kann aber auch Brom oder Iod sein.

Die 3,4-Dihydro-2H-1-benzothiopyran-3-ylmethyl- und -ethylamine der vorliegenden Erfindung können nach den allgemein anerkannten Nomenklaturregeln ebenfalls 3,4-Dihydro-2H-benzo[b]thiin-3-ylmethyl- und -ethylamine genannt werden.

Abhängig von der Art der Substituenten und der sich daraus ergebenden Anzahl asymmetrischer Kohlenstoffatome können die Verbindungen der vorliegenden Erfindung in Form einer Anzahl Racemate oder deren optischer Antipoden vorkommen. Die erfindungsgemässen Verbindungen können deshalb als Stereoisomere vorliegen, beispielsweise Diastereoisomere, Racemate, reine Enantiomere oder Mischungen dieser Stereoisomeren. Alle diese Stereoisomeren sind durch die vorliegende Erfindung umfasst.

Die Verbindungen der Formel I sind Basen und bilden leicht Säureadditionssalze. Diese Säureadditionssalze sind vorzugsweise pharmazeutisch verwendbare, ungiftige Salze, beispielsweise Salze mit starken Mineralsäuren, beispielsweise Halogenwasserstoffsäuren, z.B. Chlorwasserstoff- oder Bromwasserstoffsäure, Schwefelsäure, Phosphorsäure, Salpetersäure oder Perchlorsäure; mit aliphatischen oder aromatischen Carbonsäuren, z.B. Ameisensäure, Essigsäure, Propionsäure, Bernsteinsäure, Glykolsäure, Milchsäure, Aepfelsäure, Weinsäure, Glukonsäure, Zitronensäure, Ascorbinsäure, Maleinsäure, Fumarsäure, Hydroxymaleinsäure, Brenztraubensäure, Phenylessigsäure, Benzoesäure, 4-Aminobenzoesäure, Anthranilsäure, 4-Hydroxybenzoesäure, Salicylsäure, 4-Aminosalicylsäure, Embonsäure oder Nicotinsäure; oder mit Sulfonsäuren, z.B. Methansulfonsäure, Ethansulfonsäure, Hydroxyethansulfonsäure, Benzolsulfonsäure, p-Toluolsulfonsäure, Naphthalinsulfonsäure, Sulfanilsäure oder Cyclohexylaminosulfonsäure.

Für die Isolierung und Reinigung können auch andere als pharmazeutisch verwendbare Salze gebraucht werden. Hingegen werden nur pharmazeutisch verwendbare, ungiftige Salze therapeutisch verwendet. Diese sind deshalb bevorzugt.

Die Verbindungen der Erfindung sind in erster Linie als Serotonin-2-Rezeptor-Antagonisten und als therapeutische Wirkstoffe für die Behandlung von Störungen und Zuständen verwendbar, welche auf die Wirkung von Serotonin-2-Rezeptor-Antagonisten ansprechen, so für Störungen des zentralen Nervensystems, des Herz-Kreislaufsystems und des Magen-Darmtrakts.

Die oben genannten Eigenschaften können bei in vitro und in vivo Versuchsanordnungen gezeigt werden, in denen vorteilhafterweise Säugetiere, beispielsweise Ratten, Hunde, Affen oder isolierte Organe, Gewebeproben oder deren enzymatische Aufbereitungen verwendet werden. Die genannten Verbindungen können in vitro als Lösungen, beispielsweise bevorzugt wässrige Lösungen und in vivo entweder enteral oder parenteral, vorzugsweise oral oder intravenös, z.B. in Gelatinekapseln, als Stärkesuspensionen oder in wässrigen Lösungen, angewandt werden. Die Dosis in vitro kann zwischen ungefähr 10⁻⁶ molaren und 10⁻⁹ molaren Konzentrationen liegen. Die Dosis in vivo kann zwischen ungefähr 0,10 und 30 mg/kg/Tag, vorzugsweise zwischen ungefähr 0,50 und 20 mg/kg/Tag, insbesondere zwischen ungefähr 1,0 und 10 mg/kg/Tag betragen.

Die oben beschriebenen Verbindungen sind beispielsweise in der folgenden Versuchsanordnung aktiv, die auf Serotonin-2-Rezeptor-Antagonismus hinweist: Die Bindungseigenschaften des Serotonin-2-Rezeptors (ebenfalls als 5-Hydroxytryptamin-2- or 5HT-2-Rezeptor bezeichnet) werden im wesentlichen bestimmt, wie von Battaglia et al. in Life Sciences 33, 2011 (1983) beschrieben. Dabei wird die Hemmeigenschaft der genannten Verbindungen auf die spezifische Bindung von ³H-Ketanserin in Membran-Aufbereitungen von frontaler und parietaler Hirnrinde von männlichen Sprague-Dawley Ratten gemessen. IC₅₀-Werte, die die Konzentration an Testverbindung angeben, die benötigt wird, um 50 % ³H-Ketanserin zu verdrängen, werden durch Log-logit-Analyse der einzelnen Bindungswerte bestimmt. Die Erfindung wird durch die Verbindung von Beispiel 1 veranschaulicht, die im Serotonin-2-Rezeptor-Bindungstest einen IC₅₀-Wert von ungefähr 5 nM aufweist.

Verbindungen der Erfindung weisen ebenfalls mässigen Dopamin- und α-Rezeptor-Antagonismus auf, wie im Verdrängungstest von ³H-Spiperon im Striatum und im ³H-Prazosin-Verdrängungstest im Vorderhirn gezeigt wird.

Der Serotonin-2-Antagonismus oder die Serotonin-2-Blockade wird in vivo gezeigt, indem man die Hemmung des Kopfzuckens misst, das durch 5-Hydroxytryptophan (dem metabolischen Vorläufer von Serotonin) in der Ratte verursacht wird. Der Kopfzuck-Test zur Bestimmung des Serotonin-2-Rezeptor-Antagonismus des Zentralnervensystems in der Ratte ist in Neuropharmacology 16, 663 (1977) und in J. Pharmacol. Exp. Ther. 228, 133 (1984) beschrieben. Der Versuch wird folgendermassen ausgeführt: Männliche Wistar-Ratten (120-180 g) erhalten 18 Stunden vor dem Versuch keine Nahrung, aber Wasser nach Belieben. Alle Tiere werden mit dem peripheren Decarboxylase-Inhibitor α-Methyl-dopa-hydrazin (Carbidopa, 25 mg/kg i.p., 4,0 ml/kg) und 30 Minuten später mit 5-Hydroxytryptophan (5-HTP, 100 mg/kg s.c., 4,0 ml/kg) vorbehandelt. 90 Minuten nach der Gabe von 5-HTP werden die Ratten einzeln in Plexiglas-Beobachtungskäfige gesetzt und die Anzahl Kopfzuckungen für jedes Tier in einer Beobachtungszeit von 10 Minuten gezählt. Die zu prüfende Verbindung oder der pharmazeutische Träger allein werden entweder eine halbeStunde vor der Beobachtungszeit in einer Menge von 1,0 ml/kg i.p. oder 1, 2 oder 4 Stunden vorher in einer Menge von 10 ml/kg p.o. verabreicht. ED₅₀-Werte werden durch Probit-Analyse bestimmt. Die Erfindung wird durch die Verbindung von Beispiel 1 veranschaulicht, die im Kopfzuck-Test bei einer Dosis von ungefähr 0,8 mg/kg i.p. wirksam ist.

Weitere biologische Wirkungen der Verbindungen der Erfindung können den Eigenschaften der Serotonin-2-Rezeptor-Blockade zugeschrieben werden, beispielsweise Wirkungen auf das Zentralnervensystem und das Herz-Kreislaufsystem, die sich im Tierversuch anhand bekannter Methoden nachweisen lassen. Beispielsweise können Wirkungen, die für angstlösende Eigenschaften der Verbindungen Hinweise geben, im Standard-Cook-Davidson-Konfliktmodell in der Ratte nachgewiesen werden; eine Zunahme der Leistung deutet auf einen angstlösenden Effekt hin. Antihypertensive Eigenschaften können in der spontan hypertensiven Ratte und in anästhesierten normotensiven Hunden gezeigt werden. Antithrombotische Wirkungen können durch einen Versuch gezeigt werden, bei dem die von Serotonin verursachte Plättchenaggregation verhindert wird.

Antipsychotische (neuroleptische) Eigenschaften werden im Standard-Sidmann-Modell gezeigt. Der Versuch wird folgendermassen ausgeführt: Erwachsene männliche Pinseläffchen (Samiri sciureus, 700-1200 g) werden darauf abgerichtet, einen Hebel hinunterzupressen, um einen kurzen elektrischen Schlag um 20 Sekunden zu verzögern. Wenn das Tier nicht innerhalb der Spanne von 20 Sekunden reagiert, werden kurze (0,5 Sek.) Schläge (5 mA) alle 20 Sek. abgegeben, bis das Tier wieder den Hebel hinunterdrückt. Die gesamte Anzahl von Schlägen, die vermieden werden, und von Schlägen, die nicht vermieden werden, wird während der Versuchsdauer aufgezeichnet, welche 4 Stunden dauert. Die Affen werden 2 Tage pro Woche geprüft. Der erste Tag dient als Kontrolle der Grundlinie. Die zu prüfende Verbindung wird oral in Maisstärke 10 Min. vor Beginn des Versuchs verabreicht. Die Erfindung wird durch die Verbindung von Beispiel 1 veranschaulicht, die bei einer Dosis von 0,3 mg/kg p.o. aktiv ist. Die Verbindungen der Erfindung blockieren die Antwort auf den Sidmann-Test bei niedrigeren Dosen als solchen, die für das Auslösen des akuten diskinetischen Syndroms notwendig sind. Bei der Verbindung von Beispiel 1 wird ein ungefähres Dosisverhältnis von 3 zwischen Blockade im Sidmann-Test und Auslösung der Diskinese gefunden.

Die genannten vorteilhaften Eigenschaften bewirken, dass die Verbindungen der Erfindung in Säugetieren zur Behandlung von Störungen des Zentralnervensystems, wie Angstzustände, psychotische Störungen, Depression und Manie, aber auch zur Behandlung von Magen-Darmtraktstörungen, wie Magengeschwüren, und Herz-Kreislaufstörungen, wie Bluthochdruck und Thrombose, geeignet sind.

Die Verbindungen der vorliegenden Erfindung sind insbesondere wirksam als angstlösende und antipsychotische Wirkstoffe zur Behandlung von Angstzuständen und psychotischen Störungen, beispielsweise Schizophrenie, besonders, da sie wenig oder keine Müdigkeit oder Leistungsminderung im Wirkbereich hervorrufen und weil sie eine niedrige Tendenz aufweisen, verzögerte Diskinese und akute extrapyramidale Störungen hervorzurufen.

Besonders bevorzugt sind Verbindungen der Formel I, worin n 1 ist; X Wasserstoff, Halogen, beispielsweise Chlor, Fluor oder Brom, Niederalkyl, beispielsweise Methyl, oder Niederalkoxy, beispielsweise Methoxy, darstellt; R₁ Niederalkyl, beispielsweise Methyl oder Ethyl, ist; R₂ Niederalkyl, beispielsweise Methyl oder Ethyl, oder Aroylniederalkyl, beispielsweise Benzoyl- oder Fluorbenzoyl-ethyl, -propyl oder -butyl, ist; oder R₁ und R₂ zusammen geradkettiges Alkylen mit 4 bis 6 Kohlenstoffatomen darstellen, welches durch A substituiert ist, beispielsweise Butylen oder Pentylen, welches in β- oder γ-Stellung durch A substituiert ist; und A Wasserstoff, Hydroxymethyl, α-Hydroxyarylmethyl, beispielsweise α-Hydroxybenzyl oder α-Hydroxyfluorbenzyl, α-Hydroxydiarylmethyl, beispielsweise α-Hydroxydiphenylmethyl oder α-Hydroxydi(fluorphenyl)methyl, Niederalkoxymethyl, beispielsweise Methoxymethyl oder Ethoxymethyl, Arylniederalkoxymethyl, beispielsweise Benzyloxymethyl, Fluorbenzyloxymethyl oder 2-Phenyl- oder -Fluorphenyl-ethoxymethyl, Niederalkanoyloxymethyl, beispielsweise Acetoxymethyl oder Propionoxymethyl, Arylniederalkanoyloxymethyl, beispielsweise Phenylacetoxymethyl oder Fluorphenylacetoxymethyl, Aroyloxymethyl, beispielsweise Benzoyloxymethyl, Chlorbenzoyloxymethyl oder Fluorbenzoyloxymethyl, Niederalkanoyl, beispielsweise Acetyl, Propionyl oder Isobutyryl, Arylniederalkanoyl, beispielsweise Phenylacetyl, Fluorphenylacetyl or β-Fluorphenylpropionyl, Aroyl, beispielsweise Benzoyl, Chlorbenzoyl oder Fluorbenzoyl, Niederalkoxycarbonyl, beispielsweise Methoxy-, Ethoxy-, Isopropoxy- oder n-Butoxycarbonyl, oder Arylniederalkoxycarbonyl, beispielsweise Benzyloxycarbonyl, bedeutet; und Säureadditonssalze dieser Verbindungen.

Bevorzugt sind Verbindungen der Formel I, worin n 1 oder 2 ist, X Wasserstoff, Halogen, Niederalkyl oder Niederalkoxy bedeutet, R₁ Niederalkyl ist und R₂ Niederalkyl oder Aroylniederalkyl ist, oder R₁ und R₂ zusammen geradkettiges Butylen oder Pentylen sind; und pharmazeutisch verwendbare Säureadditionssalze dieser Verbindungen; oder Verbindungen der Formel 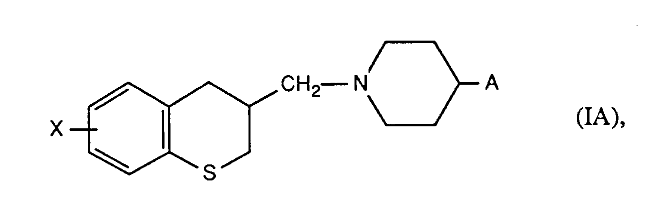
worin X Wasserstoff, Halogen, Niederalkyl oder Niederalkoxy bedeutet und A Wasserstoff, Hydroxymethyl, α-Hydroxybenzyl, α-Hydroxydiphenylmethyl, Niederalkoxymethyl, Phenylniederalkoxymethyl, Niederalkanoyloxymethyl, Phenylniederalkanoyloxymethyl, Benzoylmethyl, Niederalkanoyl, Phenylniederalkanoyl, Benzoyl, Niederalkoxycarbonyl oder Phenylniederalkoxycarbonyl bedeutet, und worin die Phenylgruppe in Phenyl, Benzyl und Benzoyl unsubstituiert oder in o-, m- oder p-Stellung, vorzugsweise in p-Stellung, durch Halogen, beispielsweise Chlor oder Fluor, substituiert ist; und pharmazeutisch verwendbare Säureadditionssalze dieser Verbindungen.

Besonders bevorzugt sind Verbindungen der Formel I, worin n 1 ist, X Wasserstoff, Halogen, Niederalkyl oder Niederalkoxy darstellt, R₁ Niederalkyl ist, R₂ Niederalkyl oder Aroylniederalkyl ist, oder R₁ und R₂ zusammen geradkettiges Butylen oder Pentylen darstellen, insbesondere solche Verbindungen, worin R₁ Niederalkyl und R₂ Aroylniederalkyl, beispielsweise p-Fluorbenzoylniederalkyl, sind, und solche Verbindungen, worin R₁ und R₂ zusammen geradkettiges Butylen darstellen; und pharmazeutisch verwendbare Säureadditionssalze dieser Verbindungen. Ganz besonders bevorzugt sind Verbindungen der Formel I, worin n 1 ist, X Fluor darstellt und in der 6-Stellung steht, R₁ Niederalkyl ist und R₂ p-Fluorbenzoylniederalkyl ist, oder R₁ und R₂ zusammen geradkettiges Butylen darstellen; und pharmazeutisch verwendbare Säureadditionssalze dieser Verbindungen.

Andere besonders bevorzugte Verbindungen sind jene der Formel IA, worin X Wasserstoff, Halogen, Niederalkyl oder Niederalkoxy darstellt und A Wasserstoff, Hydroxymethyl, α-Hydroxydi(p-fluorphenyl)methyl, Niederalkoxymethyl, p-Fluorbenzyloxymethyl, p-Fluorbenzoyloxymethyl, p-Fluorbenzoyl oder Niederalkoxycarbonyl darstellt; und pharmazeutisch verwendbare Säureadditionssalze dieser Verbindungen.

Ganz besonders bevorzugt sind Verbindungen der Formel IA, worin X Fluor darstellt und in der 6-Stellung steht und A α-Hydroxydi(p-fluorphenyl)methyl, p-Fluorbenzyloxymethyl, p-Fluorbenzoyloxymethyl oder p-Fluorbenzoyl darstellt; und pharmazeutisch verwendbare Säureadditionssalze dieser Verbindungen.

An erster Stelle sind die Verbindungen zu nennen, welche in den Beispielen beschrieben sind, insbesondere die Verbindung der Formel IA, worin X Fluor darstellt und in der 6-Stellung steht und A p-Fluorbenzoyl ist; und pharmazeutisch verwendbare Säureadditionssalze dieser Verbindung.

Die vorliegende Erfindung betrifft auch Verfahren zur Herstellung der Verbindungen der Formel I und ihrer Salze. Diese können nach an sich bekannten Methoden hergestellt werden, beispielsweise indem man
(a) eine Verbindung der Formel 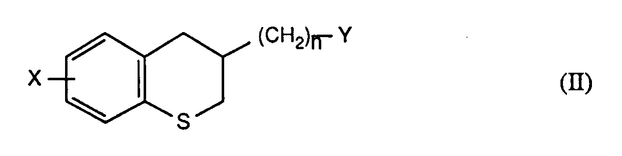 mit einem Amin R₁R₂NH, worin n, X, R₁ und R₂ die oben genannten Bedeutungen haben und Y eine Abgangsgruppe ist, umsetzt, oder
(b) eine Verbindung der Formel 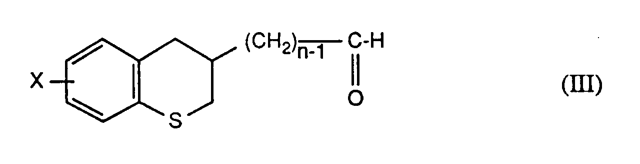 mit einem Amin R₁R₂NH, worin n, X, R₁ und R₂ die oben genannten Bedeutungen haben, unter reduzierenden Bedingungen umsetzt, oder
(c) eine Verbindung der Formel 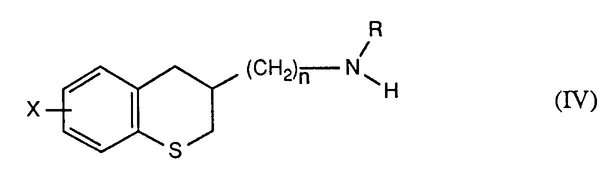 mit einem Alkylierungsmittel R₀Y, worin Y eine Abgangsgruppe, R einen Rest R₁ oder R₂ und R₀ den anderen Rest R₂ oder R₁ bedeuten, oder R Wasserstoff ist und R₀Y ein bifunktionelles Alkylierungsmittel Y-R₁-R₂-Y darstellt, worin R₁ und R₂ zusammen durch A substituiertes Alkylen von 4 bis 6 Kohlenstoffatomen bedeuten, und worin A, n, X, R₁ und R₂ die oben genannten Bedeutungen haben, umsetzt, oder
(d) eine Verbindung der Formel 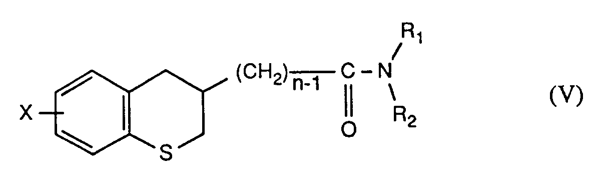 worin n, X, R₁ und R₂ die oben genannten Bedeutungen haben, reduziert, oder
(e) eine Verbindung der Formel 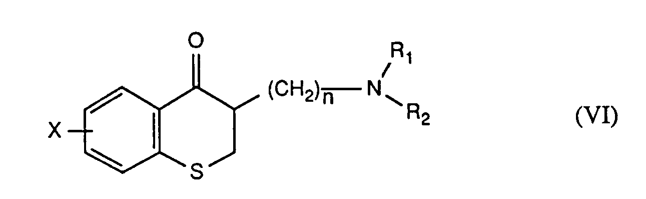 worin n, X, R₁ und R₂ die oben genannten Bedeutungen haben, reduziert,
   und, wenn erwünscht, eine erhältliche Verbindung der Formel I in eine andere Verbindung der Formel I, worin die Substituenten die oben genannten Bedeutungen haben, umwandelt, und, wenn erwünscht, eine erhältliche Verbindung der Formel I in ein Salz oder ein erhältliches Salz einer Verbindung der Formel I in die freie Verbindung oder in ein anderes Salz umwandelt, und, wenn erwünscht, eine erhältliche Mischung von Isomeren oder Racematen in die einzelnen Isomere oder Racemate auftrennt, und, wenn erwünscht, ein erhältliches Racemat in die optischen Antipoden auftrennt.

Im Teilverfahren (a) ist die Abgangsgruppe Y in Verbindungen der Formel II insbesondere durch starke anorganische oder organische Säuren verestertes Hydroxy. Beispiele von veresternden starken anorganischen Säuren sind Mineralsäuren, beispielsweise Halogenwasserstoffsäuren, wie Chlorwasserstoffsäure, Bromwasserstoffsäure oder Iodwasserstoffsäure, Schwefelsäure oder Halogenschwefelsäure, wie Fluorschwefelsäure. Beispiele von veresternden starken organischen Säuren sind Sulfonsäuren, beispielsweise eine Niederalkansulfonsäure, welche gegebenenfalls durch Halogen substituiert ist, beispielsweise Methansulfonsäure oder Trifluormethansulfonsäure, oder eine aromatische Sulfonsäure, beispielsweise eine Benzolsulfonsäure, welche gegebenenfalls durch Niederalkyl, Halogen oder Nitro substituiert ist, beispielsweise Benzolsulfonsäure, p-Toluolsulfonsäure oder p-Nitrobenzolsulfonsäure.

Die Reaktionsbedingungen in Teilprozess (a) werden vorzugsweise so gewählt, dass die Reaktion im wesentlichen als eine nucleophile Substitution zweiter Ordnung abläuft. Geeignete Lösungsmittel sind polare Lösungsmittel, z.B. Wasser, Alkohole, beispielsweise Methanol, Ethanol oder Isopropanol, oder Mischungen derselben, oder vorzugsweise dipolar aprotische Lösungsmittel, z.B. Aceton, Acetonitril, Nitromethan, Dimethylsulfoxid oder Dimethylformamid. Vorzugsweise wird eine Base zum Reaktionsgemisch gegeben, beispielsweise ein organisches Amin, insbesondere ein tertiäres Amin, wie Triethylamin, Tributylamin oder Pyridin, oder eine anorganische Base, z.B. Natrium- oder Calciumcarbonat. Die Reaktion wird in einem Temperaturbereich von -10°C bis +50°C durchgeführt, vorzugsweise ungefähr bei Raumtemperatur, gewünschtenfalls unter einer Schutzgas-Atmosphäre, z.B. unter Stickstoff.

Teilprozess (b) wird unter den üblichen Reaktionsbedingungen einer reduktiven Aminierung durchgeführt. Der Aldehyd der Formel III wird mit dem Amin in Gegenwart von Wasserstoff und eines geeigneten Hydrierkatalysators, z.B. Raney-Nickel oder Platin, in einem inerten Hydrier-Lösungsmittel, z.B. einem Alkohol, wie Ethanol, oder Essigsäureethylester, bei Raumtemperatur oder darüber, und gewünschtenfalls unter erhöhtem Wasserstoffdruck, zusammengegeben. Andernfalls wird die Mischung des Aldehyds der Formel III und des entsprechenden Amins mit Lithiumcyanborhydrid in wässriger oder alkoholischer Lösung, z.B. in Methanol, bei einem pH zwischen 4 und 7, oder mit Natriumborhydrid in wässriger oder alkoholischer Lösung, z.B. in einer Mischung aus Ethanol und Acetatpuffer, behandelt. Ameisensäure kann ebenfalls als Reduktionsmittel eingesetzt werden.

Im Teilprozess (c) hat die Abgangsgruppe Y in den Alkylierungsmitteln R₀Y eine der oben unter Teilprozess (a) beschriebenen Bedeutungen und ist beispielsweise Halogenid, wie Chlorid, Bromid oder Iodid, Hydrogensulfat, Fluorsulfat, Niederalkansulfonat, wie Methansulfonat oder Trifluormethansulfonat, oder Arylsulfonat, wie Benzolsulfonat, p-Toluolsulfonat, p-Brombenzolsulfonat oder p-Nitrobenzolsulfonat. R₀Y kann ein einwertiges Alkylierungsmittel mit der Bedeutung von R₁Y oder R₂Y oder ein zweiwertiges Alkylierungsmittel der Formel Y-R₁-R₂-Y sein. Wenn R₀Y ein zweiwertiges Alkylierungsmittel ist, kann die Reaktion mit der Aminfunktion der Verbindung der Formel IV schrittweise, mit oder ohne Isolierung des zwischenzeitlich gebildeten monoalkylierten Produktes, oder im wesentlichen in einem Schritt geschehen. Wenn ein zweiwertiges Alkylierungsmittel verwendet wird, so sind durch geeignete Verdünnungstechniken und langsame Zugabe die Bildung von Dimeren zu vermeiden oder zu verringern. Die Reaktionsbedingungen im Teilprozess (c) sind die gleichen, die oben unter Teilprozess (a) genannt sind.

Geeignete Reduktionsmittel für Teilprozess (d) sind Aluminiumhydride, z.B. Lithiumaluminiumhydrid, oder Borane, z.B. Diboran. Die Reduktion wird in etherischen Lösungsmitteln durchgeführt, z.B. in Diethylether, Tetrahydrofuran oder Dimethoxyethan, bei Temperaturen zwischen -10°C und dem Siedepunkt des Lösungsmittels, z.B. um Raumtemperatur, gewünschtenfalls unter einer inerten Schutzgas-Atmosphäre, z.B. unter Stickstoff.

Die Reduktion einer Verbindung der Formel VI in Teilprozess (e) kann in einem Schritt erreicht werden, wird aber vorzugsweise in einem mehrschrittigen Verfahren durchgeführt, wobei die Ketofunktion zu einer Alkoholgruppe reduziert, diese in eine Abgangsgruppe umgewandelt und schliesslich diese in einem weiteren Reduktionsschritt auf die Stufe einer Methylengruppe reduziert wird.

Im einschrittigen Verfahren kann die Hydrierung in Gegenwart eines Hydrierkatalysators durchgeführt werden, z.B. mit Raney-Nickel, Platin oder Palladium auf Kohle, vorzugsweise bei erhöhtem Wasserstoffdruck in einem polaren, inerten Lösungsmittel, z.B. Wasser, Ethanol, Essigsäure oder Mischungen derselben, bei Temperaturen zwischen 0°C und 100°C. Die Verbindung der Formel VI kann auch mit Hydrazin und einer starken Base, z.B. wässriger Kalilauge oder Natronlauge, in Mono- oder Diethylenglykol bei 50°C bis 150°C oder mit Kalium-tert-butanolat in Dimethylsulfoxid bei Raumtemperatur behandelt werden. Die Ketofunktion kann auch mit Zink oder Zinkamalgam in wässriger Salzsäure entfernt werden.

Im bevorzugten vielstufigen Verfahren kann das Keton der Formel VI beispielsweise mit einem Hydrid-haltigen Reduktionsmittel, wie einem Aluminiumhydrid, z.B. Lithiumaluminiumhydrid, oder einem Borhydrid, z.B. Natriumborhydrid oder Diboran, reduziert werden. Lithiumaluminiumhydrid und Diboran werden in etherischen Lösungsmitteln verwendet, z.B. in Diethylether oder Tetrahydrofuran, bei Temperaturen zwischen 0°C und dem Siedepunkt des Lösungsmittels. Natriumborhydrid wird in Wasser, einem Alkohol, z.B. Methanol oder Ethanol, oder Mischungen derselben bei Temperaturen um Raumtemperatur eingesetzt. Die Reduktion kann auch mit Aluminiumalkoxiden, z.B. Aluminiumtriisopropoxid in Gegenwart eines Ueberschusses an Isopropanol, in einem inerten Lösungsmittel, z.B. Toluol, durchgeführt werden.

Für die Umwandlung der Hydroxygruppe in eine Abgangsgruppe kann irgendeine der üblichen Veresterungsmethoden verwendet werden. Geeignete Veresterungsmittel sind z.B. Halogenwasserstoffsäure, wie Chlorwasserstoffsäure oder Bromwasserstoffsäure, Phosphorhalogenide, wie Phosphortribromid, -trichlorid oder -pentachlorid, Triphenylphosphin in Gegenwart einer Halogenquelle, z.B. Brom oder Kohlenstofftetrachlorid, Thionylchlorid oder Sulfonylchloride, die sich von Niederalkan- oder Arylsulfonsäuren, die unter Teilprozess (a) genannt sind, ableiten. Die Veresterung wird vorzugsweise in Gegenwart einer Base, z.B. eines tertiären Amins, wie Triethylamin oder Pyridin, in einem inerten Lösungsmittel, z.B. Chloroform, Methylenchlorid, Diethylether oder dergleichen, bei Temperaturen zwischen 0°C und dem Siedepunkt des Lösungsmittels durchgeführt.

Die Abgangsgruppe wird mit katalytischer Hydrierung in Gegenwart eines Hydrierkatalysators, z.B. Nickel, Platin oder Palladium auf Kohle, oder mit einem Aluminium- oder Borhydrid, z.B. Lithiumaluminiumhydrid oder Natriumborhydrid, unter den oben für diese Reduktionsmittel genannten Bedingungen durch Wasserstoff ersetzt. Andererseits kann auch ein Zinnhydrid, z.B. Tributylzinnhydrid oder Triphenylzinnhydrid, in einem inerten Lösungsmittel, z.B. Toluol, verwendet werden.

In Ausgangsmaterialien und Zwischenprodukten, welche in der beschriebenen Art und Weise zu Verbindungen der Erfindung umgesetzt werden, können vorhandene funktionelle Gruppen, wie Carbonyl-(Formyl oder Keto), Carboxy-, Amino- und Hydroxygruppen, gewünschtenfalls durch konventionelle Schutzgruppen, die in der organischen Chemie üblich sind, geschützt werden. Geschützte Carbonyl-, Carboxy-, Amino- und Hydroxygruppen sind jene, welche unter milden Bedingungen in freie Carbonyl-, Carboxy-, Amino- und Hydroxygruppen umgewandelt werden können, ohne dass das Grundgerüst zerstört wird oder andere unerwünschte Nebenreaktionen stattfinden.

Der Zweck der Einführung von Schutzgruppen ist der Schutz der funktionellen Gruppen vor unerwünschten Reaktionen mit den Reagenzien unter den Bedingungen, die für die gewünschten chemischen Umwandlungen eingesetzt werden. Der Bedarf und die Wahl der Schutzgruppen für eine spezielle Reaktion ist den Fachleuten bekannt und hängt von der Art der funktionellen Gruppe ab, die geschützt werden soll, von der Struktur und der Stabilität des Moleküls, von welchem der Substituent ein Teil ist, und von den Reaktionsbedingungen.

Die bekannten Schutzgruppen, die diese Bedingungen erfüllen, ihre Einführung und ihre Entfernung sind beispielsweise in den folgenden Standard-Werken beschrieben: J.F.W. McOmie, "Protective Groups in Organic Chemistry", Plenum Press, London, New York, 1973; T.W. Greene, "Protective Groups in Organic Synthesis", Wiley, New York, 1984; auch in "The Peptides", Band 3 (Herausgeber E. Gross und J. Meienhofer), Academic Press, London, New York, 1981; und ebenso in Houben-Weyl, "Methoden der organischen Chemie", Band 15/1, Georg Thieme Verlag, Stuttgart, 1974.

Eine Carbonylgruppe kann beispielsweise als Acetal, z.B. als Ethylen- oder Propylenacetal, oder als Thioacetal, z.B. als Propylendithioacetal, geschützt werden.

Eine Carboxygruppe kann als leicht spaltbarer Ester geschützt werden, z.B. als Benzylester, tert-Butylester oder ähnliche übliche Ester.

Ein basisches primäres oder sekundäres Amin kann als leicht spaltbares Amid geschützt werden, z.B. als Acylderivat, wie als Benzyloxycarbonyl-(Carbobenzoxy) oder als tert-Butoxycarbonyl-Derivat, oder als irgend eine andere leicht entfernbare N-Schutzgruppe.

Eine Hydroxygruppe kann als Ester geschützt werden, z.B. als Acylderivat, wie Niederalkanoyl-, Benzyloxycarbonyl- oder Niederalkoxycarbonylester, oder als Ether, z.B. als 2-Tetrahydropyranyl- oder Benzylether oder als Trimethylsilyl- oder Dimethyl-tert-butylsilylether.

In einer derart erhältlichen geschützten Verbindung der Formel I oder einem Zwischenprodukt, in welchem eine oder mehrere funktionelle Gruppen geschützt sind, können die Schutzgruppen auf bekannte Art und Weise entfernt werden, beispielsweise durch Solvolyse, z.B. Hydrolyse mit Säure, durch Reduktion, z.B. Hydrierung, oder durch Behandlung mit Oxidationsmitteln oder Fluoriden.

Salze von Verbindungen der Formel I werden auf übliche Weise erhalten, z.B. durch Behandlung mit einer equimolaren Menge oder einem leichten Ueberschuss der entsprechenden salzbildenden Säure in einem alkoholischen oder etherischen Lösungsmittel. Salze können in der üblichen Weise in freie Verbindungen umgewandelt werden, z.B. durch Behandlung mit einem geeigneten basischen Mittel. Salze können in andere Salze umgewandelt werden, indem man schrittweise zuerst die freie Verbindung und dann das andere Salz wie oben beschrieben herstellt, oder indem man das Salz mit einem Ueberschuss des entsprechenden salzbildenden Reagenzes umsetzt und wenn möglich das gewünschte Salz aus einem geeigneten Lösungsmittel kristallisiert, oder indem man das Salz mit dem entsprechenden Ionenaustauscherharz behandelt.

Angesichts der nahen Verwandtschaft zwischen freien Verbindungen und Verbindungen in der Form ihrer Salze wird, wenn immer im vorliegenden Zusammenhang von einer Verbindung gesprochen wird, auch das entsprechende Salz gemeint, unter der Bedingung, dass solches möglich und unter den Umständen passend ist.

Wenn diastereomere Mischungen der oben genannten Verbindungen oder Zwischenprodukte erhalten werden, so können diese nach bekannten Methoden in einzelne Isomere getrennt werden, beispielsweise durch fraktionierte Destillation, Kristallisation und/oder Chromatographie.

Die basischen racemischen Produkte der Formel I oder basischen Zwischenprodukte können beispielsweise in optische Antipoden aufgetrennt werden, indem man diastereomere Salze derselben trennt, z.B. durch fraktionierte Kristallisation der Salze mit d- oder l-Weinsäure, d- oder l-Dibenzoylweinsäure, d- oder l-Mandelsäure oder d- oder l-Camphersulfonsäure. Vorteilhafterweise wird das bevorzugte, aktivere Antipode der Verbindungen dieser Erfindung isoliert.

Die Ausgangsmaterialien sind bekannt oder können, wenn sie neu sind, nach bekannten Methoden hergestellt werden, wie sie in den zitierten Referenzen genannt sind oder in den folgenden Beispielen beschrieben werden.

Insbesondere werden Ausgangsmaterialien der Formel II durch eine der oben unter Teilprozess (e) genannten Veresterungsmethoden aus den entsprechenden Verbindungen, worin Y Hydroxy ist, hergestellt. Solche Alkohole sind ihrerseits durch Reduktion mit Lithiumaluminiumhydrid in Diethylether bei Raumtemperatur aus den entsprechenden Carbonsäuren erhältlich. Als Ausgangsmaterial verwendete Aldehyde der Formel III können durch Reduktion des Methyl- oder Ethylesters der entsprechenden Carbonsäuren mit Diisobutylaluminiumhydrid, aus dem Säurechlorid durch Reduktion mit Wasserstoff in Gegenwart von Palladium auf Bariumsulfat oder ferner durch Oxidation der entsprechenden Alkohole mit Chromtrioxid-Pyridin-Komplex oder Mangandioxid erhalten werden. Ausgangsmaterialien der Formel IV werden in einem Verfahren erhalten, das jenem der Teilprozesse (a) oder (d) entspricht, worin einer der Substituenten am Stickstoffatom durch Wasserstoff ersetzt ist. Carbonsäureamide der Formel V werden aus den entsprechenden Carbonsäuren durch Reaktion mit Thionylchlorid und dem entsprechenden Amin erhalten. Ausgangsmaterialien der Formel VI werden zweckdienlich durch Reaktion des mit X substituierten Benzothiopyran-4-ons entweder mit einem Formaldehyd-Amin-Komplex in einer Reaktion des Mannich-Typs oder mit einem Aminoethylhalogenid oder -sulfonat in Gegenwart einer Base erhalten.

Alle die oben genannten Reaktionen werden entsprechend den üblichen Methoden durchgeführt, in Gegenwart oder Abwesenheit von Verdünnungsmitteln, vorzugsweise in solchen, die inert sind gegenüber den verwendeten Reagenzien, in Gegenwart von Katalysatoren und/oder Kondensations- oder Neutralisierungsmitteln, und in Luft oder unter einer Schutzgas-Atmosphäre, bei niedrigen Temperaturen, Raumtemperatur oder erhöhten Temperaturen und bei Normaldruck oder erhöhtem Druck.

Die Erfindung umfasst ebenfalls Anpassungen der oben genannten Teilprozesse, worin eine Verbindung, die als Zwischenprodukt auf irgendeiner Stufe entsteht, als Ausgangsmaterial verwendet wird und die verbleibenden Reaktionsschritte ausgeführt werden, worin das Verfahren auf irgendeiner Stufe unterbrochen wird oder worin das Ausgangsmaterial unter den Reaktionsbedingungen gebildet oder in Form seines Salzes oder eines reaktiven Derivates davon verwendet wird. In den genannten Verfahren der Erfindung werden vorzugsweise jene Ausgangsmaterialien verwendet, welche zu den oben beschriebenen bevorzugten Endprodukten der Erfindung führen.

Die Erfindung betrifft ebenso neue Zwischenprodukte und Verfahren zu ihrer Herstellung.

Abhängig von der Wahl der Ausgangsmaterialien und der Verfahren können die neuen Verbindungen als Enantiomere, Racemate oder Mischungen derselben vorkommen, vorausgesetzt, solches ist möglich.

Die erfindungsgemässen Verbindungen und ihre Salze können ebenfalls in Form ihrer Hydrate erhalten werden oder andere Lösungsmittel, die zur Kristallisierung verwendet werden, einschliessen.

Die vorliegende Erfindung betrifft ebenfalls die Verwendung von Verbindungen der Formel I und ihrer pharmazeutisch verwendbaren Salze oder diese enthaltende pharmazeutische Zusammensetzungen zur Behandlung von psychotropen Störungen, wie Angstzuständen, Schizophrenie, Depression oder Manie, von Magen-Darmtrakt-Störungen, wie Magengeschwüren, und Herz-Kreislauf-Störungen, wie Bluthochdruck, in Säugetieren.

Insbesondere betrifft die Erfindung eine Methode zur Behandlung von psychotropen Störungen in Säugetieren, z.B. solchen, die auf Serotonin-2-Blockade reagieren, insbesondere Angstzuständen oder psychotischen Störungen, bei welchen eine wirksame Menge einer Verbindung der Erfindung der Formel I oder eines pharmazeutisch verwendbaren Salzes dieser Verbindung als pharmakologische Wirkstoffe verwendet werden, vorzugsweise in der Form von pharmazeutischen Zusammensetzungen.

Die Dosis der Wirksubstanz, die verabreicht wird, ist abhängig von der Art des Warmblüters (Säugetiers), des Körpergewichts, des Alters und des individuellen Zustandes, und von der Art der Verabreichung.

Eine Einheitsdosis für ein Säugetier von ungefähr 50-70 kg kann ungefähr zwischen 1 und 50 mg der aktiven Substanz enthalten.

Die vorliegende Erfindung betrifft ebenso die Verwendung der Verbindungen der Erfindung für die Herstellung von pharmazeutischen Zusammensetzungen, insbesondere von pharmazeutischen Zusammensetzungen, die die Wirkung des Serotonin-Rezeptors beeinflussen, insbesondere solchen, die die Wirkung von Serotonin-2 blockieren.

Die pharmazeutischen Zusammensetzungen entsprechend der Erfindung sind solche, die für enterale, wie orale oder rektale, und parenterale Verabreichung an Säugetiere einschliesslich des Menschen geeignet sind, insbesondere zur Behandlung von Störungen des Zentralnervensystems, wie Angstzuständen, Schizophrenie, Depession und Manie, oder zur Behandlung von Magen-Darmtrakt- oder Herz-Kreislauf-Störungen, enthaltend eine wirksame Menge der pharmakologisch aktiven Verbindung der Formel I oder ein pharmazeutisch verwendbares Salz dieser Verbindung, allein oder zusammen mit einem oder mehreren pharmazeutisch verwendbaren Trägern.

Die pharmakologisch wirksamen Verbindungen der Erfindung sind nützlich für die Herstellung von pharmazeutischen Zusammensetzungen enthaltend eine wirksame Menge derselben zusammen oder gemischt mit Trägern, die für enterale, parenterale oder topische Anwendung geeignet sind. Bevorzugt sind Tabletten und Gelatinekapseln enthaltend den aktiven Wirkstoff zusammen mit (a) Verdünnungsmitteln, z.B. Lactose, Dextrose, Sucrose, Mannit, Sorbit, Cellulose und/oder Glycin; (b) Gleitmittel, z.B. Kieselsäure, Talg, Stearinsäure, dessen Magnesium- oder Calciumsalz und/oder Polyethylenglycol; für Tabletten ebenfalls (c) Bindemittel, z.B. Magnesiumaluminiumsilikat, Stärkepaste, Gelatine, Tragacanth, Methylcellulose, Natriumcarboxymethylcellulose und/oder Polyvinylpyrrolidon; falls erwünscht, (d) Sprengmittel, z.B. Starken, Agar, Alginsäure oder dessen Natriumsalz, oder Sprudelmischungen; und/oder (e) Adsorbentien, Farbstoffe, Riechstoffe und Süssstoffe. Injizierbare Zusammensetzungen sind vorzugsweise wässrige isotonische Lösungen oder Suspensionen. Suppositorien oder oberflächlich aufzutragende Lösungen sind vorzugsweise aus Fettemulsionen oder Suspensionen hergestellt. Sie können sterilisiert sein und/oder Hilfstoffe enthalten, beispielsweise Konservier-, Stabilisier-, Netz- oder Emulgier-Mittel, Lösungsvermittler, Salze zur Regulierung des osmotischen Drucks und/oder Puffer. Die genannten pharmazeutischen Zusammensetzungen können ebenfalls andere therapeutisch wertvolle Verbindungen enthalten. Sie werden durch konventionelles Mischen, Granulieren oder Methoden des Ueberziehens hergestellt und enthalten zwischen 0,1 bis 75 %, vorzugsweise ungefähr 1 bis 50 %, des aktiven Wirkstoffs.

Geeignete Formulierungen für transdermale Anwendungen enthalten eine wirksame Menge der Verbindung der Formel I mit einem Träger. Bevorzugte Träger schliessen absorbierbare pharmazeutisch verwendbare Lösungsmittel ein, welche den Durchtritt des Wirkstoffes durch die Haut des Wirtes unterstützen. Typischerweise sind die transdermalen Vorrichtungen in der Form eines Verbandes umfassend eine Stütze, ein Reservoir enthaltend die Verbindung, gewünschtenfalls mit Trägern, gegebenenfalls eine Geschwindigkeits-bestimmende Schranke, um die Verbindung auf die Haut des Wirtes in einer kontrollierten und vorbestimmten Geschwindigkeit über eine lange Zeit abzugeben, und Mittel, um die Vorrichtung an der Haut zu befestigen.

Die folgenden Beispiele veranschaulichen die Erfindung und können nicht als Beschränkung betrachtet werden. Temperaturen werden in Celsiusgraden angegeben. Teile sind jeweils Gewichtsteile. Wenn nichts anderes gesagt wird, werden Verdampfungen unter reduziertem Druck ausgeführt, vorzugsweise zwischen 3 mbar und 100 mbar. Die Struktur der Endprodukte, Zwischenprodukte und Ausgangsmaterialien wird beispielsweise durch analytische Methoden, wie Mikroanalyse, und spektroskopische Eigenschaften, z.B. Massenspektroskopie, Infrarot-Spektroskopie oder magnetische Kernresonanz-Spektroskopie, gesichert.

### Beispiel 1: 1-[(6-Fluor-3,4-dihydro-2H-1-benzothiopyran-3-yl)methyl]-4-piperidinyl-p-fluorphenyl-keton

Zu einer Suspension von 2,178 kg (10,5 Mol) p-Fluorphenyl-4-piperidinyl-keton-hydrochlorid (R.L. Duncan et al., J. Med. Chem. 13, 1 (1970)), 1,008 kg (9,96 Mol) Triethylamin und 25 l Dimethylformamid wird eine Lösung von 3,053 kg (9,9 Mol) (6-Fluor-3,4-dihydro-2H-1-benzothiopyran-3-yl)methyliodid in 9 l Dimethylformamid unter Rühren bei 23°C während 45 Min. zugegeben. Die Suspension wird während 3 Tagen bei Raumtemperatur gerührt. Die Reaktionsmischung wird filtriert und das Filtrat bei 80°C/4 mbar eingeengt. Das erhältlich Oel wird in eine Mischung von 20 l Wasser und 24 l Essigsäureethylester gegossen, auf 5°C gekühlt und mit konzentrierter Ammoniumhydroxidlösung auf pH 10 basisch gestellt. Die organische Phase wird abgetrennt, die wässrige Lösung weiter mit Essigsäureethylester extrahiert und die vereinigten organischen Lösungen mit Wasser und Sole gewaschen, über Natriumsulfat und Aktivkohle getrocknet, filtriert und bei 50°C/4 mbar eingeengt. Der Rückstand wird mit Heptan aufgeschlämmt, filtriert, zweimal mit Heptan gewaschen, im Vakuum getrocknet und in Methylenchlorid gelöst. Unlösliches wird abfiltriert, die Lösung mit 1,27 kg Kieselgel 60 während 30 Min. gerührt, dann filtriert und bei 45°C/4 mbar eingedampft. Die Titelverbindung wird als Festkörper mit Smp. 121-124°C erhalten.

Das Hydrochlorid der Titelverbindung wird folgendermassen hergestellt: 2,24 kg (5,78 Mol) der Titelverbindung als freie Base und 28 l Ethanol werden gemischt und auf 70°C erwärmt, und es werden 0,515 l (6,15 Mol) konzentrierte Salzsäure zugegeben. Das Hydrochlorid kristallisiert bei der Abkühlung auf 12°C über Nacht. Die Festkörper werden abfiltriert, mit Ethanol und Diethylether gewaschen, im Vakuum getrocknet und ergeben das Hydrochlorid der Titelverbindung, Smp. 238-240°C (Zers.). Der Schmelzpunkt erhöht sich auf 240-243°C durch Umkristallisation aus Wasser.

Das Ausgangsmaterial wird folgendermassen hergestellt:
a) α-(p-Fluorphenylthiomethyl)acrylsäure: Zu einer Lösung von 3,293 kg (25,69 Mol) p-Fluorthiophenol in 19,3 l Methanol wird eine Lösung von 3,32 kg (86 %, 51,79 Mol) Kaliumhydroxid in 3,32 l Wasser tropfenweise unter Rühren bei 0°C zugegeben. Dann wird eine Lösung von 4,452 kg (26,98 Mol) α-Brommethylacrylsäure in 4,3 l Methanol mit solcher Geschwindigkeit zugegeben, dass die Reaktionstemperatur unterhalb 15°C bleibt. Die Mischung wird während zusätzlichen 2 Stunden bei 10°C gerührt, dann in kaltes Wasser (80 l) gegossen und mit konzentrierter Salzsäure auf pH 1 angesäuert. Das Produkt wird abfiltriert, mit Wasser gewaschen und im Vakuum getrocknet, Smp. 110-112°C.
b) 6-Fluor-3,4-dihydro-2H-1-benzothiopyran-3-carbonsäure: 1,105 kg (8,55 Mol) N,N-Diisopropylethylamin wird zu einer entgasten Mischung von 1,819 kg (8,56 Mol) α-(p-Fluorphenylthiomethyl)acrylsäure und 8,5 l o-Dichlorbenzol unter Rühren bei 30°C unter Stickstoff zugegeben. Die gebildete Lösung wird während 48 Stunden auf 165-170°C erhitzt, dann bei 70°C/4 mbar eingeengt. Der Rückstand wird in Diethylether gelöst und mit 2,5 N wässriger Natronlauge extrahiert. Die basische Lösung wird mit konzentrierter Salzsäure auf pH 1 angesäuert. Der Festkörper wird abgetrennt und in Diethylether gelöst. Die Etherlösung wird mit Wasser und Sole gewaschen, über Natriumsulfat getrocknet und im Vakuum eingedampft. Dabei wird die Titelverbindung erhalten, Smp. 98-102°C.
c) 6-Fluor-3,4-dihydro-2H-1-benzothiopyran-3-methanol: Eine Lösung von 2,13 kg (10,03 Mol) 6-Fluor-3,4-dihydro-2H-1-benzothiopyran-3-carbonsäure in 9,2 l Tetrahydrofuran wird während 3 Stunden zu 11,568 Mol Boran in 22,5 l Tetrahydrofuran zugegeben, wobei die Reaktionstemperatur unter 15°C gehalten wird. Die Mischung wird über Nacht bei Raumtemperatur gerührt, dann mit 1 l 50%-iger wässriger Essigsäure hydrolysiert und bei 50°C/4 mbar eingeengt. Der Rückstand wird mit 6 l Wasser verdünnt und mit konzentrierter Ammoniumhydroxid-Lösung auf pH 10 basisch gestellt. Das Produkt wird mit Diethylether extrahiert, die Etherlösung mit Sole gewaschen, über Natriumsulfat getrocknet und eingedampft. Die Titelverbindung wird als ein Oel erhalten.
d) (6-Fluor-3,4-dihydro-2H-1-benzothiopyran-3-yl)methyl-methansulfonat: Eine Lösung von 2,687 kg (23,45 Mol) Methansulfonylchlorid in 3,3 l Methylenchlorid wird während 2 Stunden zu einer Lösung von 4,215 kg (21,26 Mol) 6-Fluor-3,4-dihydro-2H-1-benzothiopyran-3-methanol und 2,374 kg (23,46 Mol) Triethylamin in 34 l Methylenchlorid zugegeben, wobei die Reaktionstemperatur unter 15°C gehalten wird. Das Gemisch wird über Nacht bei Raumtemperatur gerührt, dann unter Kühlen mit 26 l Wasser hydrolysiert. Die Methylenchlorid-Lösung wird abgetrennt, mit Wasser und Sole gewaschen, über Natriumsulfat getrocknet und eingedampft. Der ölige Rückstand wird in 6 l Essigsäureethylester gelöst, filtriert und mit 6 l Heptan behandelt. Die Titelverbindung kristallisiert aus. Sie wird abfiltriert, mit Essigsäureethylester/Heptan 1:3 gewaschen und getrocknet, Smp. 70,5-72,5°C. Aufschlämmen in wasserfreiem Ethylether erhöht den Schmelzpunkt auf 72-74°C.
e) (6-Fluor-3,4-dihydro-2H-1-benzothiopyran-3-yl)methyliodid: 5.53 kg (36,89 Mol) Natriumiodid werden zu einer Lösung von 3,361 kg (12,16 Mol) (6-Fluor-3,4-dihydro-2H-1-benzothiopyran-3-yl)methyl-methansulfonat in 38,6 l Aceton unter Rühren bei 15°C zugegeben. Es erfolgt eine leicht exotherme Reaktion. Die Suspension wird unter einer Stickstoffatmosphäre während 6 Stunden unter Rückfluss erhitzt, dann über Nacht bei Raumtemperatur gerührt und bei 35°C/4 mbar eingeengt. Der Festkörper wird in 22 l Wasser suspendiert und mit 4 x 10 l Diethylether extrahiert. Die vereinigten etherischen Lösungen werden mit Sole gewaschen, über Natriumsulfat und Aktivkohle G-60 getrocknet und im Vakuum eingedampft. Dabei wird die Titelverbindung als ein Oel erhalten.

### Beispiel 2: Die folgenden Verbindungen werden unter Verwendung des Verfahrens des Beispiels 1 erhalten:

a) 6-Brom-3-dimethylaminomethyl-3,4-dihydro-2H-1-benzothiopyran-hydrochlorid, Smp. 210-222°C, aus (6-Brom-3,4-dihydro-2H-1-benzothiopyran-3-yl)methyliodid, Smp. 69-71°C, und Dimethylamin. Das Iodid wird seinerseits aus der entsprechenden 6-Brom-3,4-dihydro-2H-1-benzothiopyran-3-carbonsäure, Smp. 197-203°C, durch Reduktion zum Alkohol, Mesylierung und Behandlung mit Natriumiodid hergestellt.
b) 6-Brom-3-piperidinomethyl-3,4-dihydro-2H-1-benzothiopyran-hydrochlorid, Smp. 212-215°C.
c) 1-[(6-Fluor-3,4-dihydro-2H-1-benzothiopyran-3-yl)methyl]piperidin-4-carbonsäureethylester, Smp. 64-68°C.

### Beispiel 3: 3-Dimethylaminomethyl-3,4-dihydro-2H-1-benzothiopyran

Zu einer Suspension von 2 g Lithiumaluminiumhydrid in 100 ml Ether werden 2 g N,N-Dimethyl-3,4-dihydio-2H-1-benzothiopyran-3-carbonsäureamid gelöst in 50 ml Ether tropfenweise zugegeben. Die Mischung wird während 3 Stunden unter Rückfluss erhitzt und das überschüssige Reagens durch langsame tropfenweise Zugabe von Wasser unter Kühlung zersetzt. Die Reaktionsmischung wird filtriert, im Vakuum eingeengt und mit ethanolischer HCl angesäuert, wobei das Hydrochlorid der Titelverbindung erhalten wird, Smp. 183-187°C.

Das Ausgangsmaterial wird folgendermassen hergestellt:
N,N-Dimethyl-3,4-dihydro-2H-1-benzothiopyran-3-carbonsäureamid: Eine Mischung von 5,0 g 3,4-Dihydro-2H-1-benzothiopyran-3-carbonsäure und 20 ml Thionylchlorid wird während 30 Min. auf 90°C erhitzt. Das überschüssige Reagens wird im Vakuum entfernt der Rückstand in 100 ml Methylenchlorid gelöst und langsam mit 5 ml Dimethylamin, gelöst in 10 ml Methylenchlorid, behandelt. Nach 10 Min. bei Raumtemperatur wird die Reaktionsmischung mit Wasser gewaschen, über Magnesiumsulfat getrocknet und das Lösungsmittel im Vakuum entfernt, wobei die Titelverbindung als Oel erhalten wird.

### Beispiel 4: Die folgenden Verbindungen werden analog Beispiel 3 hergestellt:

a) 3-Dimethylaminomethyl-6-methoxy-3,4-dihydro-2H-1-benzothiopyran-hydrochlorid, Smp. 184-188°C, aus 6-Methoxy-3,4-dihydro-2H-1-benzothiopyran-3-carbonsäure, Smp. 150-154°C.
b) 3-Dimethylaminomethyl-6-fluor-3,4-dihydro-2H-1-benzothiopyran-hydrochlorid, Smp. 185-196°C, aus 6-Fluor-3,4-dihydro-2H-1-benzothiopyran-3-carbonsäure, Beispiel 1b.
c) 3-Dimethylaminomethyl-8-methoxy-3,4-dihydro-2H-1-benzothiopyran-hydrochlorid, Smp. 221-223°C, aus 8-Methoxy-3,4-dihydro-2H-1-benzothiopyran-3-carbonsäure, Smp. 140-144°C.
d) 3-Dimethylaminomethyl-8-fluor-3,4-dihydro-2H-1-benzothiopyran-hydrochlorid, Smp. 214-219°C, aus 8-Fluor-3,4-dihydro-2H-1-benzothiopyran-3-carbonsäure, Smp. 147-149°C.
e) 3-Diethylaminomethyl-6-fluor-3,4-dihydro-2H-1-benzothiopyran-hydrochlorid, Smp. 146-149°C.
f) 6-Fluor-3-pyrrolidinomethyl-3,4-dihydro-2H-1-benzothiopyran-hydrochlorid, Smp. 162-176°C.
g) 6-Fluor-3-piperidinomethyl-3,4-dihydro-2H-1-benzothiopyran-hydrochlorid, Smp. 195-199°C.

### Beispiel 5: 6-Fluor-3-(2-piperidinoethyl)-3,4-dihydro-2H-1-benzothiopyran

Das Hydrochlorid der Titelverbindung, Smp. 160-162°C, wird durch Reduktion des entsprechenden Carbonsäureamids analog Beispiel 3 durch Reduktion mit Lithiumaluminiumhydrid hergestellt. Das Amid wird aus der Carbonsäure mit Thionylchlorid und Piperidin hergestellt.

Das Ausgangsmaterial wird folgendermassen hergestellt:
6-Fluor-3,4-dihydro-2H-1-benzothiopyran-3-ylessigsäure: Eine Mischung von 3,8 g 6-Fluor-3-iodmethyl-3,4-dihydro-2H-1-benzothiopyran und 30 ml 0,5 M Lithiumcyanid in Dimethylformamid wird während 24 Stunden bei Raumtemperatur gerührt und dann in Wasser gegossen. Das Produkt wird mit Ether extrahiert. Nach dem Trocknen über Magnesiumsulfat wird das Lösungsmittel im Vakuum entfernt. Der Rückstand wird während 6 Stunden in einer Mischung von 20 ml Essigsäure und 20 ml 12 N HCl unter Rückfluss gekocht. Das Lösungsmittel wird im Vakuum entfernt, der Rückstand in 1 N NaOH gelöst und mit Ether gewaschen. Die wässrige Phase wird mit 3 N HCl angesäuert und das Produkt mit Ether extrahiert. Das Lösungsmittel wird über Magnesiumsulfat getrocknet und im Vakuum entfernt. Der Rückstand wird in Ether/Hexan aufgeschlämmt und ergibt die Titelverbindung, Smp. 125-135°C.

### Beispiel 6: 3-(N-[(6-Fluor-3,4-dihydro-2H-1-benzothiopyran-3-yl)methyl]-N-methylamino)propyl-p-fluorphenyl-keton

Eine Mischung von 400 mg 6-Fluor-3-methylaminomethyl-3,4-dihydro-2H-1-benzothiopyran, 600 mg γ-Chlor-p-fluorbutyrophenon, 450 mg Natriumiodid und 500 mg Natriumbicarbonat in 15 ml Dimethylformamid wird unter Rühren während 40 Min. auf 100°C erhitzt. Das Reaktionsgemisch wird in Wasser gegossen und das Produkt mit Ether extrahiert. Nach Trocknen über Magnesiumsulfat wird das Lösungsmittel im Vakuum entfernt und der Rückstand durch Chromatographie an Kieselgel mit Ether/Methylenchlorid als Laufmittel gereinigt. Die Hauptfraktion wird mit ethanolischer HCl und Ether behandelt und ergibt das Hydrochlorid der Titelverbindung, Smp. 140-144°C.

Das Ausgangsmaterial 6-Fluor-3-methylaminomethyl-3,4-dihydro-2H-1-benzothiopyran wird aus dem entsprechenden Iodid und Methylamin analog Beispiel 1 hergestellt. Es wird ohne weitere Reinigung verwendet.

### Beispiel 7: 6-Fluor-3-(4-[hydroxymethyl]piperidinomethyl)-3,4-dihydro-2H-1-benzothiopyran

Eine Lösung von 3,0 g 1-[(6-Fluor-3,4-dihydro-2H-1-benzothiopyran-3-yl)methyl]piperidin-4-carbonsäureethylester in 30 ml Ether wird tropfenweise unter Rühren und Kühlen zu einer Mischung von 2,5 g Lithiumaluminiumhydrid in 120 ml Ether zugegeben. Nach 30 Min. bei Raumtemperatur wird das überschüssige Reagens mit Wasser zersetzt, das Reaktionsgemisch filtriert und das Lösungsmittel im Vakuum entfernt. Der Rückstand wird mit ethanolischer HCl und Ether behandelt und ergibt das Hydrochlorid der Titelverbindung, Smp. 132-136°C.

### Beispiel 8: 6-Fluor-3-[4-(p-fluorbenzyloxymethyl)piperidinomethyl]-3,4-dihydro-2H-1-benzothiopyran

Zu einer Lösung von 600 mg 6-Fluor-3-(4-[hydroxymethyl]piperidinomethyl)-3,4-dihydro-2H-1-benzothiopyran in 20 ml Dimethylsulfoxid werden hintereinander 300 mg Natriumhydrid und 0,5 ml p-Fluorbenzylchlorid zugegeben. Das Reaktionsgemisch wird während 6 Stunden gerührt. Das Reaktionsgemisch wird mit Wasser verdünnt und das Produkt mit Ether extrahiert. Das Lösungsmittel wird über Magnesiumsulfat getrocknet, im Vakuum entfernt und der Rückstand über Kieselgel mit Ether/Methylenchlorid als Laufmittel chromatogriphisch gereinigt. Die Verbindung wird mit ethanolischer HCl und Ether behandelt und ergibt das Hydrochlorid der Titelverbindung, Smp. 176-180°C.

### Beispiel 9: (1-[(6-Fluor-3,4-dihydro-2H-1-benzothiopyran-3-yl)methyl]-4-piperidinyl)methyl-p-fluorbenzoat

Zu einer Lösung von 400 mg 6-Fluor-3-(4-[hydroxymethyl]piperidinomethyl)-3,4-dihydro-2H-1-benzothiopyran und 200 mg Triethylamin in 10 ml Methylenchlorid werden 221 mg p-Fluorbenzoylchlorid zugegeben. Nach 30 Min. bei Raumtemperatur wird das Reaktionsgemisch mit 1 N Natriumhydroxid gewaschen, über Magnesiumsulfat getrocknet und das Lösungsmittel im Vakuum entfernt. Der Rückstand wird in Ether/Hexan aufgeschlämmt und ergibt die Titelverbindung, Smp. 113-114°C.

### Beispiel 10: (1-[(6-Fluor-3,4-dihydro-2H-1-benzothiopyran-3-yl)methyl]-4-piperidinyl)di(p-fluorphenyl)methanol

Zu einer Lösung von 1,75 g 4-Bromfluorbenzol in 30 ml Tetrahydrofuran (THF) werden bei -78°C 4 ml 2,6 M n-Butyllithium in Hexan von -78°C zugegeben. Nach 15 Min. bei -78°C wird eine Lösung von 3,37 g 1-[(6-Fluor-3,4-dihydro-2H-1-benzothiopyran-3-yl)methyl]piperidin-4-carbonsäureethylester in 10 ml THF tropfenweise zugegeben. Das Reaktionsgemisch lässt man während 30 Min. auf 0°C aufwärmen, dann wird es mit wässriger Essigsäure abgeschreckt. Die Produkte werden mit Ether extrahiert Das Lösungsmittel wird über Magnesiumsulfat getrocknet und im Vakuum entfernt. Der gebildete teniäre Alkohol und das Keton werden durch Chromatographie an Kieselgel mit Ether/Hexan/Triethylamin als Laufmittel aufgetrennt. Fraktionen enthaltend Alkohol werden zusammengegeben und das Oel mit heisser Fumarsäure in Ethanol behandelt, wobei das Monofumarat der Titelverbindung gebildet wird, Smp. 217-220°C. Die Keton-Fraktionen werden in Ether/Hexan aufgeschlämmt und ergeben die Verbindung von Beispiel 1, 1-[(6-Fluor-3,4-dihydro-2H-1-benzothiopyran-3-yl)methyl]-4-piperidinyl-p-fluorphenyl-keton, Smp. 123-125°C.

### Beispiel 11: Kapseln

1000 Kapseln, enthaltend je 5 mg der Wirksubstanz, werden gemäss folgender Formel hergestellt:
1-[(6-Fluor-3,4-dihydro-2H-1-benzothiopyran-3-yl)methyl]-4-piperidinyl-p-fluorphenylketon: 5,0 g
Lactose: 207,0 g
Modifizierte Stärke: 80,0 g
Magnesiumstearat: 3,0 g
Alle pulverigen Substanzen werden durch ein Sieb mit Oeffnungen von 0,6 mm gedrückt. Dann wird die Wirksubstanz in einen geeigneten Mixer gegeben und zuerst mit Magnesiumstearat, dann mit Lactose und der Stärke gemischt, bis eine homogene Mischung entsteht. Hartgelatinekapseln Nr. 2 werden mit je 315 mg dieser Mischung gefüllt, wobei eine Kapselfüllmaschine verwendet wird.

Analog werden Kapseln enthaltend 2-50 mg der anderen beschriebenen Verbindungen hergestellt.

### Beispiel 12: Tabletten

10000 Tabletten enthaltend je 10 mg Wirksubstanz werden folgendermassen hergestellt:
1-[(6-Fluor-3,4-dihydro-2H-1-benzothiopyran-3-yl)methyl]-4-piperidinyl-p-fluorphenylketon: 100 g
Lactose: 2535 g
Maisstärke: 125 g
Polyethylenglykol 6000: 150 g
Talgpulver: 150 g
Magnesiumstearat: 40 g
gereinigtes Wasser: q.s.
Alle pulverförmigen Substanzen werden durch ein Sieb mit Oeffnungen von 0,6 mm gepresst. Dann werden Wirksubstanz, Lactose, Talg, Magnesiumstearat und die Hälfte der Stärke in einem geeigneten Mixer gemischt. Die andere Hälfte der Stärke wird in 65 ml Wasser suspendiert und die Suspension zu einer siedenden Lösung von Polyethylenglykol in 260 ml Wasser zugegeben. Die gebildete Paste wird zum Pulver zugegeben und granuliert, falls nötig mit einer zusätzlichen Menge Wasser. Das Granulat wird über Nacht bei 35°C getrocknet, auf einem Sieb mit Oeffnungen von 1,2 mm gebrochen und in Tabletten gepresst, wobei konkave Stanzen, die obere mit einer Halbierungslinie, verwendet werden.

Analog werden Tabletten enthaltend 2-50 mg einer anderen der in den vorstehenden Beispielen beschriebenen Wirksubstanz hergestellt.

## Patentansprüche (Patentansprüche für folgende(n) Vertragsstaat(en): AT, BE, CH, DE, DK, FR, GB, IT, LI, LU, NL, SE)

1. Verbindungen der Formel 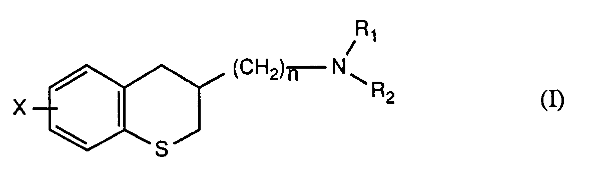 worin n 1 oder 2 ist, X Wasserstoff, Halogen, Niederalkyl, Hydroxy oder Niederalkoxy bedeutet, R₁ Niederalkyl ist, R₂ durch A substituiertes Niederalkyl ist, oder R₁ und R₂ zusammen Alkylen mit 4 bis 6 Kohlenstoffatomen darstellen, das durch A substituiert ist, und worin A Wasserstoff, Hydroxymethyl, α-Hydroxyarylmethyl, α-Hydroxydiarylmethyl, Niederalkoxymethyl, Arylniederalkoxymethyl, Niederalkanoyloxymethyl, Arylniederalkanoyloxymethyl, Aroyloxymethyl, Niederalkanoyl, Arylniederalkanoyl, Aroyl, Niederalkoxycarbonyl oder Arylniederalkoxycarbonyl bedeutet; und Salze dieser Verbindungen.

2. Verbindungen gemäss Anspruch 1 der Formel I, worin n 1 ist, X Wasserstoff, Halogen, Niederalkyl oder Niederalkoxy darstellt, R₁ Niederalkyl ist, R₂ Niederalkyl oder Aroylniederalkyl ist, oder R₁ und R₂ zusammen geradkettiges Alkylen mit 4-6 Kohlenstoffatomen darstellen, welches durch A substituiert ist, und A Wasserstoff, Hydroxymethyl, α-Hydroxyarylmethyl, α-Hydroxydiarylmethyl, Niederalkoxymethyl, Arylniederalkoxymethyl, Niederalkanoyloxymethyl, Arylniederalkanoyloxymethyl, Aroyloxymethyl, Niederalkanoyl, Arylniederalkanoyl, Aroyl, Niederalkoxycarbonyl oder Arylniederalkoxycarbonyl bedeutet; und Säureadditionssalze dieser Verbindungen.

3. Verbindungen gemäss Anspruch 1 der Formel I, worin n 1 oder 2 ist, X Wasserstoff, Halogen, Niederalkyl oder Niederalkoxy bedeutet, R₁ Niederalkyl ist und R₂ Niederalkyl oder Aroylniederalkyl ist, oder R₁ und R₂ zusammen geradkettiges Butylen oder Pentylen sind; und pharmazeutisch verwendbare Säureadditionssalze dieser Verbindungen.

4. Verbindungen gemäss Anspruch 3 der Formel I, worin n 1 ist, X Wasserstoff, Halogen, Niederalkyl oder Niederalkoxy darstellt, R₁ Niederalkyl ist und R₂ Aroylniederalkyl ist; und pharmazeutisch verwendbare Säureadditionssalze dieser Verbindungen.

5. Verbindungen gemäss Anspruch 3 der Formel I, worin n 1 ist, X Wasserstoff, Halogen, Niederalkyl oder Niederalkoxy darstellt und R₁ und R₂ zusammen geradkettiges Butylen darstellen; und pharmazeutisch verwendbare Säureadditionssalze dieser Verbindungen.

6. Verbindungen gemäss Anspruch 3 der Formel I, worin n 1 ist, X Fluor darstellt und in der 6-Stellung steht, R₁ Niederalkyl ist und R₂ p-Fluorbenzoylniederalkyl ist, oder R₁ und R₂ zusammen geradkettiges Butylen darstellen; und pharmazeutisch verwendbare Säureadditionssalze dieser Verbindungen.

7. Verbindungen gemäss Anspruch 1 der Formel 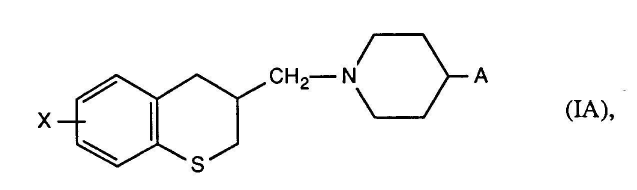 worin X Wasserstoff, Halogen, Niederalkyl oder Niederalkoxy bedeutet und A Wasserstoff, Hydroxymethyl, α-Hydroxybenzyl, α-Hydroxydiphenylmethyl, Niederalkoxymethyl, Phenylniederalkoxymethyl, Niederalkanoyloxymethyl, Phenylniederalkanoyloxymethyl, Benzoylmethyl, Niederalkanoyl, Phenylniederalkanoyl, Benzoyl, Niederalkoxycarbonyl oder Phenylniederalkoxycarbonyl bedeutet, und worin die Phenylgruppe in Phenyl, Benzyl und Benzoyl unsubstituiert oder in o-, m- oder p-Stellung durch Halogen substituiert ist; und pharmazeutisch verwendbare Säureadditionssalze dieser Verbindungen.

8. Verbindungen gemäss Anspruch 7 der Formel IA, worin X Wasserstoff, Halogen, Niederalkyl oder Niederalkoxy darstellt und A Wasserstoff, Hydroxymethyl, α-Hydroxydi(p-fluorphenyl)methyl, Niederalkoxymethyl, p-Fluorbenzyloxymethyl, p-Fluorbenzoyloxymethyl, p-Fluorbenzoyl oder Niederalkoxycarbonyl darstellt; und pharmazeutisch verwendbare Säureadditionssalze dieser Verbindungen.

9. Verbindungen gemäss Anspruch 7 der Formel IA, worin X Fluor darstellt und in der 6-Stellung steht und A α-Hydroxydi(p-fluorphenyl)methyl, p-Fluorbenzyloxymethyl, p-Fluorbenzoyloxymethyl oder p-Fluorbenzoyl darstellt; und pharmazeutisch verwendbare Säureadditionssalze dieser Verbindungen.

10. Die Verbindung gemäss Anspruch 7 der Formel IA, worin X Fluor darstellt und in der 6-Stellung steht und A p-Fluorbenzoyl ist; und pharmazeutisch verwendbare Säureadditionssalze dieser Verbindung.

11. Verfahren zur Herstellung der Verbindungen der Formel I gemäss Anspruch 1 und ihrer Salze, dadurch gekennzeichnet, dass man
(a) eine Verbindung der Formel 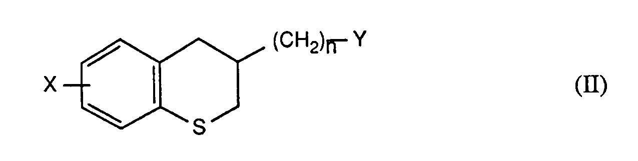 mit einem Amin R₁R₂NH, worin n, X, R₁ und R₂ die oben genannten Bedeutungen haben und Y eine Abgangsgruppe ist, umsetzt, oder
(b) eine Verbindung der Formel 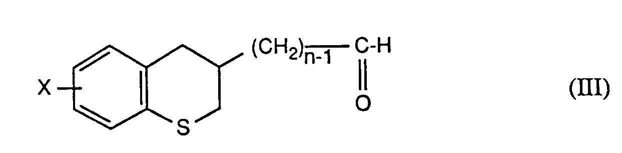 mit einem Amin R₁R₂NH, worin n, X, R₁ und R₂ die oben genannten Bedeutungen haben, unter reduzierenden Bedingungen umsetzt, oder
(c) eine Verbindung der Formel 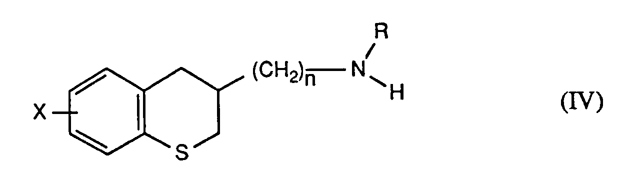 mit einem Alkylierungsmittel R₀Y, worin Y eine Abgangsgruppe, R einen Rest R₁ oder R₂ und R₀ den anderen Rest R₂ oder R₁ bedeuten, oder R Wasserstoff ist und R₀Y ein bifunktionelles Alkylierungsmittel Y-R₁-R₂-Y darstellt, worin R₁ und R₂ zusammen durch A substituiertes Alkylen von 4 bis 6 Kohlenstoffatomen bedeuten, und worin A, n, X, R₁ und R₂ die oben genannten Bedeutungen haben, umsetzt, oder
(d) eine Verbindung der Formel 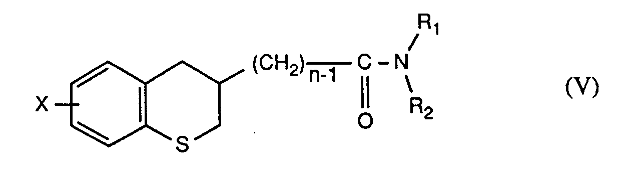 worin n, X, R₁ und R₂ die oben genannten Bedeutungen haben, reduziert, oder
(e) eine Verbindung der Formel 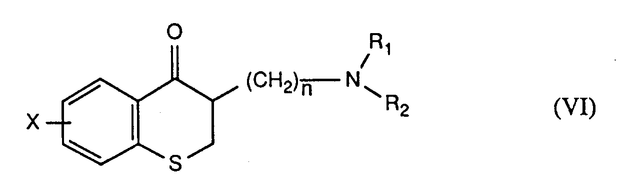 worin n, X, R₁ und R₂ die oben genannten Bedeutungen haben, reduziert,
und, wenn erwünscht, eine erhältliche Verbindung der Formel I in eine andere Verbindung der Formel I, worin die Substituenten die oben genannten Bedeutungen haben, umwandelt, und, wenn erwünscht, eine erhältliche Verbindung der Formel I in ein Salz oder ein erhältliches Salz einer Verbindung der Formel I in die freie Verbindung oder in ein anderes Salz umwandelt, und, wenn erwünscht, eine erhältliche Mischung von Isomeren oder Racematen in die einzelnen Isomere oder Racemate auftrennt, und, wenn erwünscht, ein erhältliches Racemat in die optischen Antipoden auftrennt.

12. Pharmazeutische Zusammensetzungen enthaltend eine Verbindung gemäss Anspruch 1 oder ein Salz davon und einen pharmazeutisch verwendbaren Träger.

13. Verwendung von Verbindungen gemäss Anspruch 1 oder ihren Salzen zur Herstellung von pharmazeutischen Zusammensetzungen zur Behandlung von Störungen des Zentralnervensystems, des Magen-Darmtrakts oder des Herz-Kreislaufsystems.

14. Eine Verbindung gemäss einem der Ansprüche 1 bis 10 oder ein pharmazeutisch verwendbares Säureadditionssalz davon zur Anwendung in einem Verfahren zur therapeutischen oder prophylaktischen Behandlung des menschlichen oder tierischen Körpers.

## Patentansprüche (Patentansprüche für folgende(n) Vertragsstaat(en): ES, GR)

1. Verfahren zur Herstellung von Verbindungen der Formel 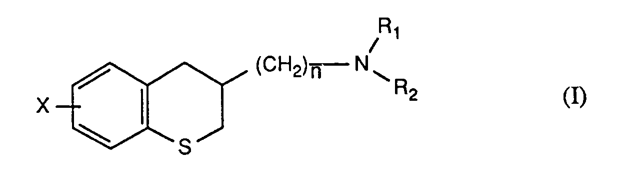 worin n 1 oder 2 ist, X Wasserstoff, Halogen, Niederalkyl, Hydroxy oder Niederalkoxy bedeutet, R₁ Niederalkyl ist, R₂ durch A substituiertes Niederalkyl ist, oder R₁ und R₂ zusammen Alkylen mit 4 bis 6 Kohlenstoffatomen darstellen, das durch A substituiert ist, und worin A Wasserstoff, Hydroxymethyl, α-Hydroxyarylmethyl, α-Hydroxydiarylmethyl, Niederalkoxymethyl, Arylniederalkoxymethyl, Niederalkanoyloxymethyl, Arylniederalkanoyloxymethyl, Aroyloxymethyl, Niederalkanoyl, Arylniederalkanoyl, Aroyl, Niederalkoxycarbonyl oder Arylniederalkoxycarbonyl bedeutet, und von Salzen dieser Verbindungen, dadurch gekennzeichnet, dass man
(a) eine Verbindung der Formel 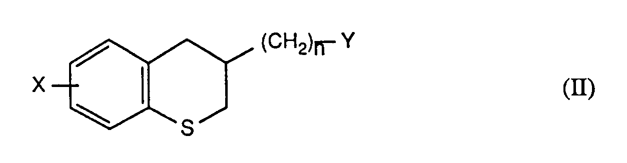 mit einem Amin R₁R₂NH, worin n, X, R₁ und R₂ die oben genannten Bedeutungen haben und Y eine Abgangsgruppe ist, umsetzt, oder
(b) eine Verbindung der Formel 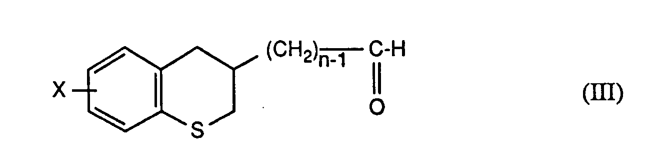 mit einem Amin R₁R₂NH, worin n, X, R₁ und R₂ die oben genannten Bedeutungen haben, unter reduzierenden Bedingungen umsetzt, oder
(c) eine Verbindung der Formel 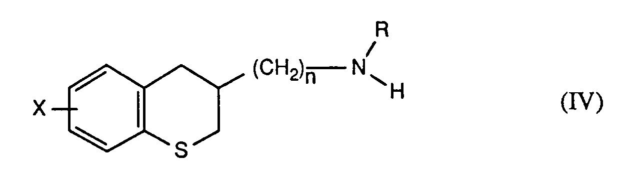 mit einem Alkylierungsmittel R₀Y, worin Y eine Abgangsgruppe, R einen Rest R₁ oder R₂ und R₀ den anderen Rest R₂ oder R₁ bedeuten, oder R Wasserstoff ist und R₀Y ein bifunktionelles Alkylierungsmittel Y-R₁-R₂-Y darstellt, worin R₁ und R₂ zusammen durch A substituiertes Alkylen von 4 bis 6 Kohlenstoffatomen bedeuten, und worin A, n, X, R₁ und R₂ die oben genannten Bedeutungen haben, umsetzt, oder
(d) eine Verbindung der Formel 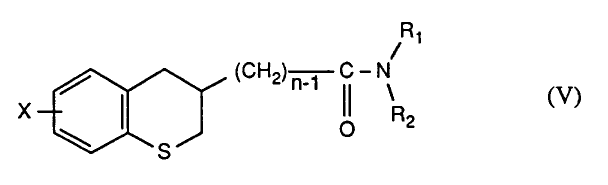 worin n, X, R₁ und R₂ die oben genannten Bedeutungen haben, reduziert, oder
(e) eine Verbindung der Formel 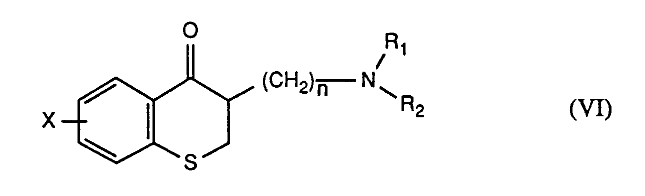 worin n, X, R₁ und R₂ die oben genannten Bedeutungen haben, reduziert,
und, wenn erwünscht, eine erhältliche Verbindung der Formel I in eine andere Verbindung der Formel I, worin die Substituenten die oben genannten Bedeutungen haben, umwandelt, und, wenn erwünscht, eine erhältliche Verbindung der Formel I in ein Salz oder ein erhältliches Salz einer Verbindung der Formel I in die freie Verbindung oder in ein anderes Salz umwandelt, und, wenn erwünscht, eine erhältliche Mischung von Isomeren oder Racematen in die einzelnen Isomere oder Racemate auftrennt, und, wenn erwünscht, ein erhältliches Racemat in die optischen Antipoden auftrennt.

2. Verfahren gemäss Anspruch 1 zur Herstellung von Verbindungen der Formel I, worin n 1 ist, X Wasserstoff, Halogen, Niederalkyl oder Niederalkoxy darstellt, R₁ Niederalkyl ist, R₂ Niederalkyl oder Aroylniederalkyl ist, oder R₁ und R₂ zusammen geradkettiges Alkylen mit 4-6 Kohlenstoffatomen darstellen, welches durch A substituiert ist, und A Wasserstoff, Hydroxymethyl, α-Hydroxyarylmethyl, α-Hydroxydiarylmethyl, Niederalkoxymethyl, Arylniederalkoxymethyl, Niederalkanoyloxymethyl, Arylniederalkanoyloxymethyl, Aroyloxymethyl, Niederalkanoyl, Arylniederalkanoyl, Aroyl, Niederalkoxycarbonyl oder Arylniederalkoxycarbonyl bedeutet; und von Säureadditionssalzen dieser Verbindungen.

3. Verfahren gemäss Anspruch 1 zur Herstellung von Verbindungen der Formel I, worin n 1 oder 2 ist, X Wasserstoff, Halogen, Niederalkyl oder Niederalkoxy bedeutet, R₁ Niederalkyl ist und R₂ Niederalkyl oder Aroylniederalkyl ist, oder R₁ und R₂ zusammen geradkettiges Butylen oder Pentylen sind; und von pharmazeutisch verwendbaren Säureadditionssalzen dieser Verbindungen.

4. Verfahren gemäss Anspruch 3 zur Herstellung von Verbindungen der Formel I, worin n 1 ist, X Wasserstoff, Halogen, Niederalkyl oder Niederalkoxy darstellt, R₁ Niederalkyl ist und R₂ Aroylniederalkyl ist; und von pharmazeutisch verwendbaren Säureadditionssalzen dieser Verbindungen.

5. Verfahren gemäss Anspruch 3 zur Herstellung von Verbindungen der Formel I, worin n 1 ist, X Wasserstoff, Halogen, Niederalkyl oder Niederalkoxy darstellt und R₁ und R₂ zusammen geradkettiges Butylen darstellen; und von pharmazeutisch verwendbaren Säureadditionssalzen dieser Verbindungen.

6. Verfahren gemäss Anspruch 3 zur Herstellung von Verbindungen der Formel I, worin n 1 ist, X Fluor darstellt und in der 6-Stellung steht, R₁ Niederalkyl ist und R₂ p-Fluorbenzoylniederalkyl ist, oder R₁ und R₂ zusammen geradkettiges Butylen darstellen; und von pharmazeutisch verwendbaren Säureadditionssalzen dieser Verbindungen.

7. Verfahren gemäss Anspruch 1 zur Herstellung von Verbindungen der Formel 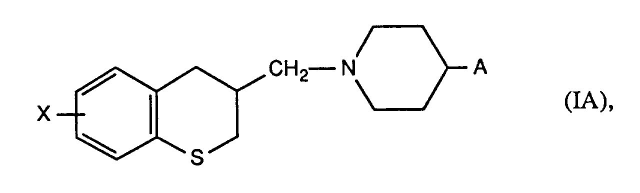 worin X Wasserstoff, Halogen, Niederalkyl oder Niederalkoxy bedeutet und A Wasserstoff, Hydroxymethyl, α-Hydroxybenzyl, α-Hydroxydiphenylmethyl, Niederalkoxymethyl, Phenylniederalkoxymethyl, Niederalkanoyloxymethyl, Phenylniederalkanoyloxymethyl, Benzoylmethyl, Niederalkanoyl, Phenylniederalkanoyl, Benzoyl, Niederalkoxycarbonyl oder Phenylniederalkoxycarbonyl bedeutet, und worin die Phenylgruppe in Phenyl, Benzyl und Benzoyl unsubstituiert oder in o-, m- oder p-Stellung durch Halogen substituiert ist; und von pharmazeutisch verwendbaren Säureadditionssalzen dieser Verbindungen.

8. Verfahren gemäss Anspruch 7 zur Herstellung von Verbindungen der Formel IA, worin X Wasserstoff, Halogen, Niederalkyl oder Niederalkoxy darstellt und A Wasserstoff, Hydroxymethyl, α-Hydroxydi(p-fluorphenyl)methyl, Niederalkoxymethyl, p-Fluorbenzyloxymethyl, p-Fluorbenzoyloxymethyl, p-Fluorbenzoyl oder Niederalkoxycarbonyl darstellt; und von pharmazeutisch verwendbaren Säureadditionssalzen dieser Verbindungen.

9. Verfahren gemäss Anspruch 7 zur Herstellung von Verbindungen der Formel IA, worin X Fluor darstellt und in der 6-Stellung steht und A α-Hydroxydi(p-fluorphenyl)methyl, p-Fluorbenzyloxymethyl, p-Fluorbenzoyloxymethyl oder p-Fluorbenzoyl darstellt; und von pharmazeutisch verwendbaren Säureadditionssalzen dieser Verbindungen.

10. Verfahren gemäss Anspruch 7 zur Herstellung der Verbindung der Formel IA, worin X Fluor darstellt und in der 6-Stellung steht und A p-Fluorbenzoyl ist; und von pharmazeutisch verwendbaren Säureadditionssalzen dieser Verbindung.

11. Verfahren zur Herstellung pharmazeutischer Zusammensetzungen, dadurch gekennzeichnet, dass man eine gemäss Anspruch 1 hergestellte Verbindung oder ein Salz davon mit einem pharmazeutisch verwendbaren Träger mischt.

12. Verwendung einer Verbindung gemäss einem der Ansprüche 1 bis 10 oder eines pharmazeutisch verwendbaren Säureadditionssalzes davon zur Herstellung von pharmazeutischen Zusammensetzungen zur Behandlung von Störungen des Zentralnervensystems, des Magen-Darmtrakts oder des Herz-Kreislaussystems.

## Claims (Claims for the following Contracting State(s): AT, BE, CH, DE, DK, FR, GB, IT, LI, LU, NL, SE)

1. A compound of the formula 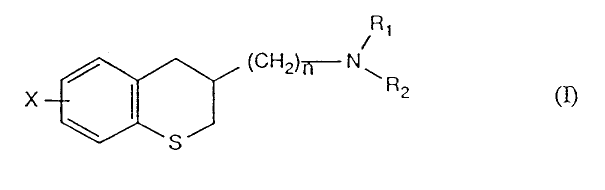 wherein n is one or two, X represents hydrogen, halogen, lower alkyl, hydroxy or lower alkoxy, R₁ is lower alkyl, R₂ is lower alkyl substituted by A, or R₁ and R₂ together represent alkylene of 4 to 6 carbon atoms substituted by A, and wherein A is hydrogen, hydroxymethyl, α-hydroxyarylmethyl, α-hydroxydiarylmethyl, lower alkoxymethyl, aryl-lower alkoxymethyl, lower alkanoyloxymethyl, aryl-lower alkanoyloxymethyl, aroyloxymethyl, lower alkanoyl, aryl-lower alkanoyl, aroyl, lower alkoxycarbonyl or aryl-lower alkoxycarbonyl; or a salt of such a compound.

2. A compound according to claim 1 of formula I wherein n is one, X represents hydrogen, halogen, lower alkyl or lower alkoxy, R₁ is lower alkyl, R₂ is lower alkyl or aroyl-lower alkyl, or R₁ and R₂ together represent straight-chain alkylene of 4 to 6 carbon atoms substituted by A, and A is hydrogen, hydroxymethyl, α-hydroxyarylmethyl, α-hydroxydiarylmethyl, lower alkoxymethyl, aryl-lower alkoxymethyl, lower alkanoyloxymethyl, aryl-lower alkanoyloxymethyl, aroyloxymethyl, lower alkanoyl, aryl-lower alkanoyl, aroyl, lower alkoxycarbonyl or aryl-lower alkoxycarbonyl; or an acid addition salt of such a compound.

3. A compound according to claim 1 of formula I wherein n is one or two, X represents hydrogen, halogen, lower alkyl or lower alkoxy, R₁ is lower alkyl and R₂ is lower alkyl or aroyl-lower alkyl, or R₁ and R₂ together are straight-chain butylene or pentylene; or a pharmaceutically acceptable acid addition salt of such a compound.

4. A compound according to claim 3 of formula I wherein n is one, X represents hydrogen, halogen, lower alkyl or lower alkoxy, R₁ is lower alkyl and R₂ is aroyl-lower alkyl; or a pharmaceutically acceptable acid addition salt of such a compound.

5. A compound according to claim 3 of formula I wherein n is one, X represents hydrogen, halogen, lower alkyl or lower alkoxy, and R₁ and R₂ together represent straight-chain butylene; or a pharmaceutically acceptable acid addition salt of such a compound.

6. A compound according to claim 3 of formula I wherein n is one, X represents fluoro and is located in the 6-position, and R₁ is lower alkyl and R₂ is p-fluorobenzoyl-lower alkyl, or R₁ and R₂ together represent straight-chain butylene; or a pharmaceutically acceptable acid addition salt of such a compound.

7. A compound according to claim 1 of the formula 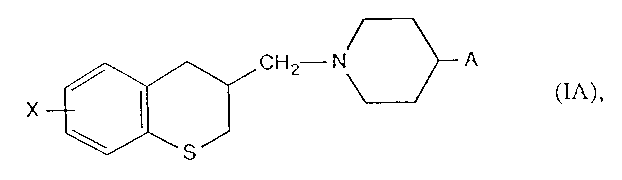 wherein X represents hydrogen, halogen, lower alkyl or lower alkoxy, and A is hydrogen, hydroxymethyl, α-hydroxybenzyl, α-hydroxydiphenylmethyl, lower alkoxymethyl, phenyl-lower alkoxymethyl, lower alkanoyloxymethyl, phenyl-lower alkanoyloxymethyl, benzoylmethyl, lower alkanoyl, phenyl-lower alkanoyl, benzoyl, lower alkoxycarbonyl or phenyl-lower alkoxycarbonyl, and wherein the phenyl group in phenyl, benzyl and benzoyl is unsubstituted or substituted by halogen in the o-, m- or p-position; or a pharmaceutically acceptable acid addition salt of such a compound.

8. A compound according to claim 7 of formula IA wherein X represents hydrogen, halogen, lower alkyl or lower alkoxy, and A is hydrogen, hydroxymethyl, α-hydroxydi(p-fluorophenyl)methyl, lower alkoxymethyl, p-fluorobenzyloxymethyl, p-fluorobenzoyloxymethyl, p-fluorobenzoyl or lower alkoxycarbonyl; or a pharmaceutically acceptable acid addition salt of such a compound.

9. A compound according to claim 7 of formula IA wherein X represents fluoro and is located in the 6-position, and A represents α-hydroxydi(p-fluorophenyl)methyl, p-fluorobenzyloxymethyl, p-fluorobenzoyloxymethyl or p-fluorobenzoyl; or a pharmaceutically acceptable acid addition salt of such a compound.

10. The compound according to claim 7 of formula IA wherein X represents fluoro and is located in the 6-position, and A represents p-fluorobenzoyl; or a pharmaceutically acceptable acid addition salt of such a compound.

11. A process for the manufacture of a compound of formula I according to claim 1 or a salt thereof, comprising
(a) reacting a compound of the formula 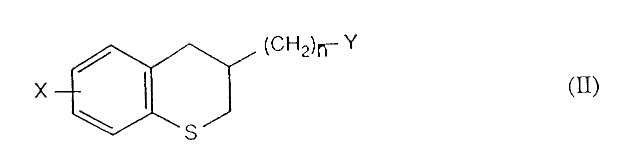 with an amine R₁R₂NH, wherein n, X, R₁ and R₂ have the meaning as previously defined and Y is a leaving group, or
(b) reacting a compound of the formula 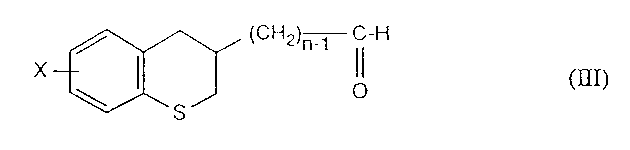 with an amine R₁R₂NH under reducing conditions, wherein n, X, R₁ and R₂ have the meaning as previously defined, or
(c) reacting a compound of the formula 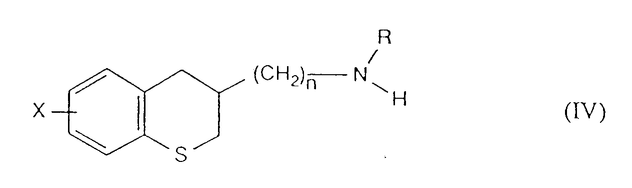 with an alkylating agent R₀Y, wherein Y is a leaving group, R is a radical R₁ or R₂ and R₀ represents the other radical R₂ or R₁, respectively, or R is hydrogen and R₀Y represents a bifunctional alkylating agent Y-R₁-R₂-Y wherein R₁ and R₂ together represent alkylene of 4 to 6 carbon atoms substituted by A, and wherein A, n, X, R₁ and R₂ have the meaning as previously defined, or
(d) reducing a compound of the formula 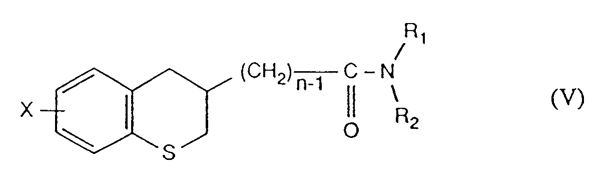 wherein n, X, R₁ and R₂ have the meaning as previously defined, or
(e) reducing a compound of the formula 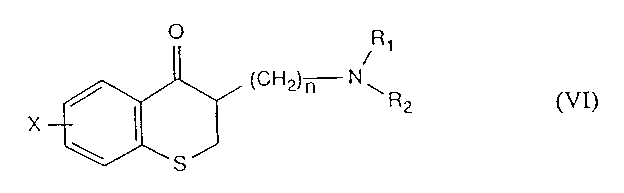 wherein n, X, R₁ and R₂ have the meaning as previously defined,
and, if desired, converting an obtainable compound of formula I into another compound of formula I wherein the substituents have the meaning as previously defined, and, if desired, converting an obtainable compound of formula I into a salt or converting an obtainable salt of a compound of formula I into the free compound or into another salt, and, if desired, separating an obtainable mixture of isomers or racemates into the single isomers or racemates, and, if desired, resolving an obtainable racemate into the optical antipodes.

12. A pharmaceutical composition comprising a compound according to claim 1 or a salt thereof and a pharmaceutically acceptable carrier.

13. The use of a compound according to claim 1 or a salt thereof in the manufacture of pharmaceutical compositions for the treatment of disorders of the central nervous system, the gastrointestinal tract or the cardiovascular system.

14. A compound according to any one of claims 1 to 10 or a pharmaceutically acceptable acid addition salt thereof for use in a method for the therapeutic or prophylactic treatment of the human or animal body.

## Claims (Claims for the following Contracting State(s): ES, GR)

1. A process for the manufacture of a compound of the formula 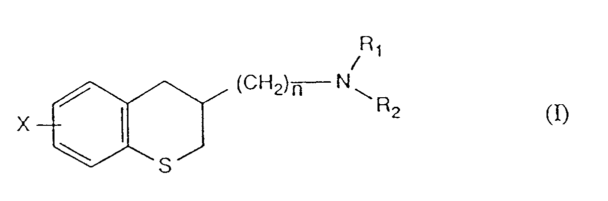 wherein n is one or two, X represents hydrogen, halogen, lower alkyl, hydroxy or lower alkoxy, R₁ is lower alkyl, R₂ is lower alkyl substituted by A, or R₁ and R₂ together represent alkylene of 4 to 6 carbon atoms substituted by A, and wherein A is hydrogen, hydroxymethyl, α-hydroxyarylmethyl, α-hydroxydiarylmethyl, lower alkoxymethyl, aryl-lower alkoxymethyl, lower alkanoyloxymethyl, aryl-lower alkanoyloxymethyl, aroyloxymethyl, lower alkanoyl, aryl-lower alkanoyl, aroyl, lower alkoxycarbonyl or aryl-lower alkoxycarbonyl, or a salt of such a compound, comprising
(a) reacting a compound of the formula 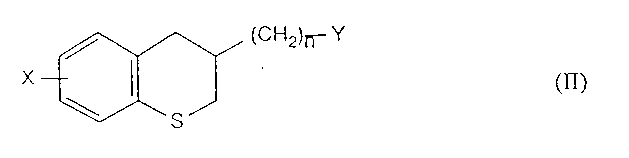 with an amine R₁R₂NH, wherein n, X, R₁ and R₂ have the meaning as previously defined and Y is a leaving group, or
(b) reacting a compound of the formula 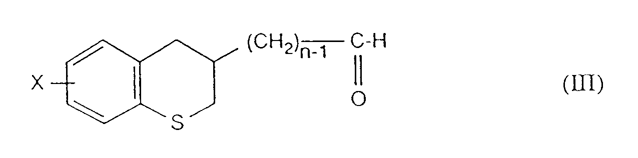 with an amine R₁R₂NH under reducing conditions, wherein n, X, R₁ and R₂ have the meaning as previously defined, or
(c) reacting a compound of the formula 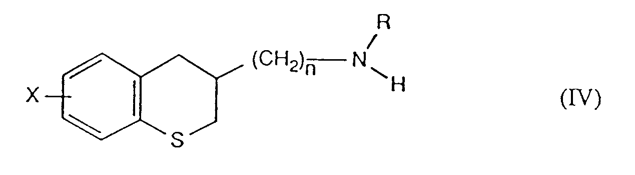 with an alkylating agent R₀Y, wherein Y is a leaving group, R is a radical R₁ or R₂ and R₀ represents the other radical R₂ or R₁, respectively, or R is hydrogen and R₀Y represents a bifunctional alkylating agent Y-R₁-R₂-Y wherein R₁ and R₂ together represent alkylene of 4 to 6 carbon atoms substituted by A, and wherein A, n, X, R₁ and R₂ have the meaning as previously defined, or
(d) reducing a compound of the formula 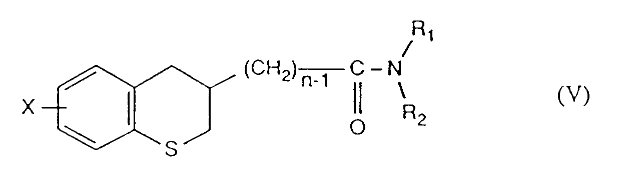 wherein n, X, R₁ and R₂ have the meaning as previously defined, or
(e) reducing a compound of the formula 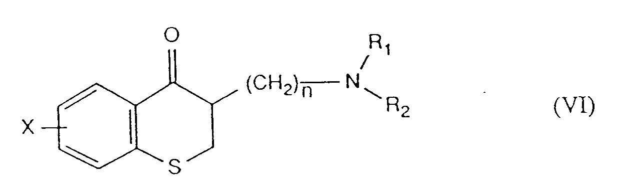 wherein n, X, R₁ and R₂ have the meaning as previously defined,
and, if desired, converting an obtainable compound of formula I into another compound of formula I wherein the substituents have the meaning as previously defined, and, if desired, converting an obtainable compound of formula I into a salt or converting an obtainable salt of a compound of formula I into the free compound or into another salt, and, if desired, separating an obtainable mixture of isomers or racemates into the single isomers or racemates, and, if desired, resolving an obtainable racemate into the optical antipodes.

2. A process according to claim 1 for the manufacture of a compound of formula I wherein n is one, X represents hydrogen, halogen, lower alkyl or lower alkoxy, R₁ is lower alkyl, R₂ is lower alkyl or aroyl-lower alkyl, or R₁ and R₂ together represent straight-chain alkylene of 4 to 6 carbon atoms substituted by A, and A is hydrogen, hydroxymethyl, α-hydroxyarylmethyl, α-hydroxydiarylmethyl, lower alkoxymethyl, aryl-lower alkoxymethyl, lower alkanoyloxymethyl, aryl-lower alkanoyloxymethyl, aroyloxymethyl, lower alkanoyl, aryl-lower alkanoyl, aroyl, lower alkoxycarbonyl or aryllower alkoxycarbonyl; or an acid addition salt of such a compound.

3. A process according to claim 1 for the manufacture of a compound of formula I wherein n is one or two, X represents hydrogen, halogen, lower alkyl or lower alkoxy, R₁ is lower alkyl and R₂ is lower alkyl or aroyl-lower alkyl, or R₁ and R₂ together are straight-chain butylene or pentylene; or a pharmaceutically acceptable acid addition salt of such a compound.

4. A process according to claim 3 for the manufacture of a compound of formula I wherein n is one, X represents hydrogen, halogen, lower alkyl or lower alkoxy, R₁ is lower alkyl and R₂ is aroyl-lower alkyl; or a pharmaceutically acceptable acid addition salt of such a compound.

5. A process according to claim 3 for the manufacture of a compound of formula I wherein n is one, X represents hydrogen, halogen, lower alkyl or lower alkoxy, and R₁ and R₂ together represent straight-chain butylene; or a pharmaceutically acceptable acid addition salt of such a compound.

6. A process according to claim 3 for the manufacture of a compound of formula I wherein n is one, X represents fluoro and is located in the 6-position, and R₁ is lower alkyl and R₂ is p-fluorobenzoyl-lower alkyl, or R₁ and R₂ together represent straight-chain butylene; or a pharmaceutically acceptable acid addition salt of such a compound.

7. A process according to claim 1 for the manufacture of a compound of the formula 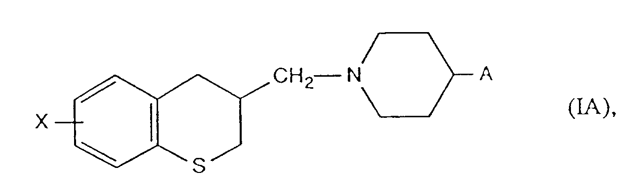 wherein X represents hydrogen, halogen, lower alkyl or lower alkoxy, and A is hydrogen, hydroxymethyl, α-hydroxybenzyl, α-hydroxydiphenylmethyl, lower alkoxymethyl, phenyl-lower alkoxymethyl, lower alkanoyloxymethyl, phenyl-lower alkanoyloxymethyl, benzoylmethyl, lower alkanoyl, phenyl-lower alkanoyl, benzoyl, lower alkoxycarbonyl or phenyl-lower alkoxycarbonyl, and wherein the phenyl group in phenyl, benzyl and benzoyl is unsubstituted or substituted by halogen in the o-, m- or p-position; or a pharmaceutically acceptable acid addition salt of such a compound.

8. A process according to claim 7 for the manufacture of a compound of formula IA wherein X represents hydrogen, halogen, lower alkyl or lower alkoxy, and A is hydrogen, hydroxymethyl, α-hydroxydi(p-fluorophenyl)methyl, lower alkoxymethyl, p-fluorobenzyloxymethyl, p-fluorobenzoyloxymethyl, p-fluorobenzoyl or lower alkoxycarbonyl; or a pharmaceutically acceptable acid addition salt of such a compound.

9. A process according to claim 7 for the manufacture of a compound of formula IA wherein X represents fluoro and is located in the 6-position, and A represents α-hydroxydi(p-fluorophenyl)methyl, p-fluorobenzyloxymethyl, p-fluorobenzoyloxymethyl or p-fluorobenzoyl; or a pharmaceutically acceptable acid addition salt of such a compound.

10. A process according to claim 7 for the manufacture of a compound of formula IA wherein X represents fluoro and is located in the 6-position, and A represents p-fluorobenzoyl; or a pharmaceutically acceptable acid addition salt of such a compound.

11. A process for the manufacture of a pharmaceutical composition which comprises mixing a compound manufactured in accordance with claim 1 or a salt thereof with a pharmaceutically acceptable carrier.

12. The use of a compound according to any one of claims 1 to 10 or a pharmaceutically acceptable acid addition salt thereof in the manufacture of pharmaceutical compositions for the treatment of disorders of the central nervous system, the gastrointestinal tract or the cardiovascular system.

## Revendications (Revendications pour l'(les) Etat(s) contractant(s) suivant(s): AT, BE, CH, DE, DK, FR, GB, IT, LI, LU, NL, SE)

1. Composés de formule 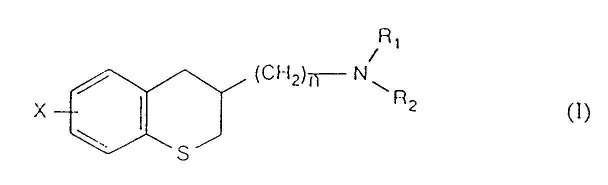 dans laquelle n vaut 1 ou 2, X représente un hydrogène, halogène, alkyle inférieur, hydroxy ou alcoxy inférieur, R₁ représente un alkyle inférieur, R₂ un alkyle inférieur substitué par A, où R₁ et R₂ représentent ensemble un alkylène en C₄ à C₆, qui est substitué par A et où A représente un hydrogène, hydroxyméthyle, α-hydroxyarylméthyle, α-hydroxydiarylméthyle, alcoxy inférieur méthyle, arylalcoxy inférieur méthyle, alcanoyloxy inférieur méthyle, arylalcanoyloxy inférieur méthyle, aroyloxyméthyle, alcanoyle inférieur, arylalcanoyle inférieur, aroyle, alcoxy inférieur carbonyle ou arylalcoxy inférieur carbonyle, et les sels de ces composés.

2. Composés selon la revendication 1 de formule I, où n vaut 1, X représente un hydrogène, halogène, alkyle inférieur ou alcoxy inférieur, R₁ représente un alkyle inférieur, R₂ un alkyle inférieur ou un aroyl-alkyle inférieur, où R₁ et R₂ représentent ensemble un alkylène à chaîne droite en C₄ à C₆, qui est substitué par A et où A représente un hydrogène, hydroxyméthyle, α-hydroxyarylméthyle, α-hydroxydiarylméthyle, alcoxy inférieur méthyle, arylalcoxy inférieur méthyle, alcanoyloxy inférieur méthyle, arylalcanoyloxy inférieur méthyle, aroyloxyméthyle, alcanoyle inférieur, arylalcanoyle inférieur, aroyle, alcoxy inférieur carbonyle ou arylalcoxy inférieur carbonyle, et les sels d'addition d'acides de ces composés.

3. Composés selon la revendication 1 de formule I, dans laquelle n vaut 1 ou 2, X représente un hydrogène, halogène, alkyle inférieur ou alcoxy inférieur, R₁ est un alkyle inférieur, et R₂ est un alkyle inférieur ou un aroylalkyle inférieur, ou R₁ et R₂ représentent ensemble un butylène ou pentylène à chaîne droite; et les sels d'addition d'acides pharmaceutiquement acceptables de ces composés.

4. Composés selon la revendication 3 de formule I, dans laquelle n vaut 1, X représente un hydrogène, halogène, alkyle inférieur ou alcoxy inférieur, R₁ est un alkyle inférieur et R₂ est un aroylalkyle inférieur; et les sels d'addition d'acides pharmaceutiquement acceptables de ces composés.

5. Composés selon la revendication 3 de formule I, dans laquelle n vaut 1, X représente un hydrogène, halogène, alkyle inférieur ou alcoxy inférieur et R₁ et R₂ représentent ensemble un butylène à chaîne droite; et les sels d'addition d'acides pharmaceutiquement acceptables de ces composés.

6. Composés selon la revendication 3 de formule I, dans laquelle n vaut 1, X représente un fluor et est en position 6, R₁ est un alkyle inférieur et R₂ est un p-fluorobenzoylalkyle inférieur, ou R₁ et R₂ représentent ensemble un butylène à chaîne droite; et les sels d'addition d'acides pharmaceutiquement acceptables de ces composés.

7. Composés selon la revendication 1 de formule 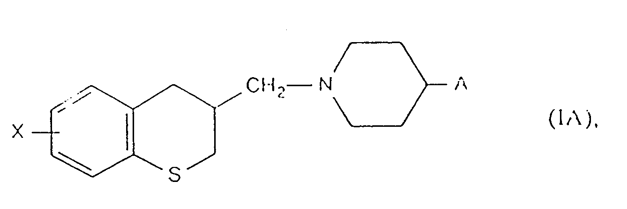 dans laquelle X représente un hydrogène, halogène, alkyle inférieur ou alcoxy inférieur et A représente un hydrogène, hydroxyméthyle, α-hydroxybenzyle, α-hydroxydiphénylméthyle, alcoxy inférieur méthyle, phénylalcoxy inférieur-méthyle, alcanoyloxy inférieur méthyle, phénylalcanoyloxy inférieur méthyle, benzoyle méthyle, alcanoyle inférieur, phénylalcanoyle inférieur, benzoyle, alcoxy inférieur carbonyle, ou phénylalcoxy inférieur carbonyle, et où le groupe phényle dans phényle, benzyle et benzoyle est non substitué ou substitué en position o, m ou p par un halogène; et les sels d'addition d'acides pharmaceutiquement acceptables de ces composés.

8. Composés selon la revendication 7 de formule IA, dans laquelle X représente un hydrogène, halogène, alkyle inférieur ou alcoxy inférieur, et A représente un hydrogène, hydroxyméthyle, α-hydroxydi(p-fluorophényl)méthyle, alcoxy inférieur méthyle, p-fluorobenzyloxyméthyle, p-fluorobenzoyloxyméthyle, p-fluorobenzoyle ou alcoxy inférieur carbonyle; et les sels d'addition d'acides pharmaceutiquement acceptables de ces composés.

9. Composés selon la revendication 7 de formule IA, dans laquelle X représente un fluor et est en position 6 et A représente un α-hydroxydi-(p-fluorophényl)méthyle, p-fluorobenzyloxyméthyle, p-fluorobenzoyloxyméthyle ou p-fluorobenzoyle; et les sels d'addition d'acides pharmaceutiquement acceptables de ces composés.

10. Le composé selon la revendication 7 de formule IA, dans laquelle X représente un fluor et est en position 6 et A représente un p-fluorobenzoyle; et les sels d'addition d'acides pharmaceutiquement acceptables de ce composé.

11. Procédé de préparation des composés de formule I selon la revendication 1 et de leurs sels, caractérisé en ce qu'on procède
(a) en faisant réagir un composé de formule 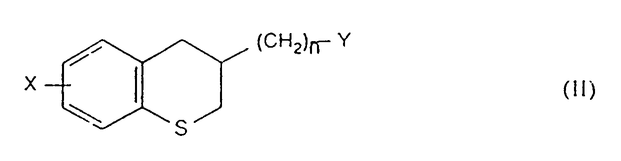 avec une amine R₁R₂NH, où n, X, R₁ et R₂ ont les significations données ci-dessus et Y représente un groupe sortant, ou
(b) en faisant réagir dans des conditions réductrices un composé de formule 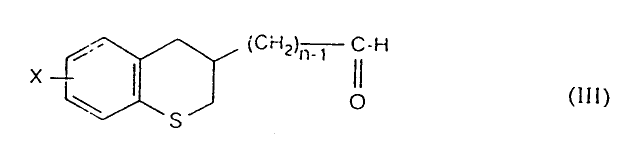 avec une amine R₁R₂NH, où n, X, R₁ et R₂ ont les significations données ci-dessus, ou
(c) en faisant réagir un composé de formule 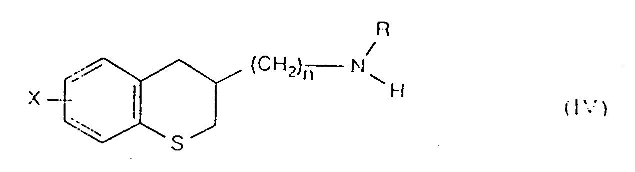 avec un agent alkylant R_{O}Y, où Y représente un groupe sortant, R un radical R₁ ou R₂ et R₀ l'autre radical R₂ ou R₁, ou bien R est un hydrogène et R₀Y est un agent alkylant bifonctionnel Y-R₁-R₂-Y, où R₁ et R₂ représentent ensemble un alkylène en C₄ à C₆ substitué par A, et où A, n, X, R₁ et R₂ ont les significations données ci-dessus, ou
(d) en réduisant un composé de formule 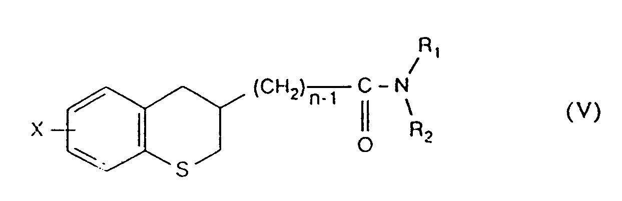 dans laquelle n, X, R₁ et R₂ ont les significations données ci-dessus, ou
(e) en réduisant un composé de formule 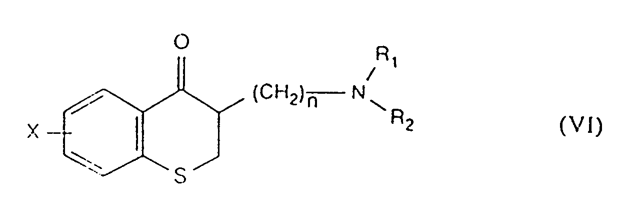 dans laquelle n, X, R₁ et R₂ ont les significations données ci-dessus,
et, si nécessaire en transformant un composé de formule I obtenu en un autre composé de formule I, où les substituants ont les significations données ci-dessus, et, si on le désire, en transformant un composé de formule I obtenu en un sel ou un sel d'un composé de formule I obtenu en le composé libre ou en un autre sel, et, si on le désire, en séparant un mélange d'isomères ou de racémates obtenu en les isomères ou racémates isolés et, si on le désire, en séparant un racémate obtenu en les antipodes optiques.

12. Compositions pharmaceutiques contenant un composé selon la revendication 1 ou un de ses sels et un support pharmaceutiquement acceptable.

13. Application de composés selon la revendication 1 ou de leurs sels à la préparation de compositions pharmaceutiques pour le traitement des désordres du système nerveux central, de l'appareil gastrointestinal ou du système circulatoire cardiaque.

14. Composé selon l'une des revendications 1 à 10 ou un de ses sels d'addition d'acides pharmaceutiquement acceptables pour application dans un procédé de traitement thérapeutique ou prophylactique du corps humain ou animal.

## Revendications (Revendications pour l'(les) Etat(s) contractant(s) suivant(s): ES, GR)

1. Procédé de préparation de composés de formule 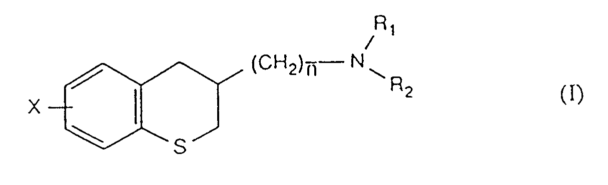 dans laquelle n vaut 1 ou 2, X représente un hydrogène, halogène, alkyle inférieur, hydroxy ou alcoxy inférieur, R₁ représente un alkyle inférieur, R₂ un alkyle inférieur substitué par A, ou R₁ et R₂ représentent ensemble un alkylène en C₄ à C₆, qui est substitué par A et où A représente un hydrogène, hydroxyméthyle, α-hydroxyarylméthyle, α-hydroxydiarylméthyle, alcoxy inférieur méthyle, arylalcoxy inférieur méthyle, alcanoyloxy inférieur méthyle, arylalcanoyloxy inférieur méthyle, aroyloxyméthyle, alcanoyle inférieur, arylalcanoyle inférieur, aroyle, alcoxy inférieur carbonyle ou arylalcoxy inférieur carbonyle, et des sels de ces composés, caractérisé en ce qu'on procède
(a) en faisant réagir un composé de formule 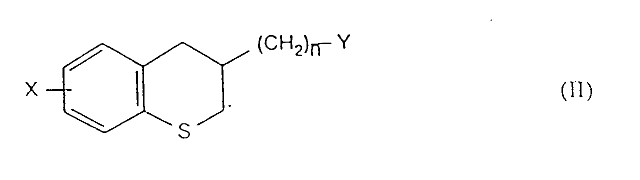 avec une amine R₁R₂NH, où n, X, R₁ et R₂ ont les significations données ci-dessus et Y représente un groupe sortant, ou
(b) en faisant réagir dans des conditions réductrices un composé de formule 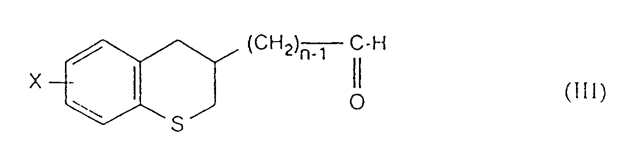 avec une amine R₁R₂NH, où n, X, R₁ et R₂ ont les significations données ci-dessus, ou
(c) en faisant réagir un composé de formule 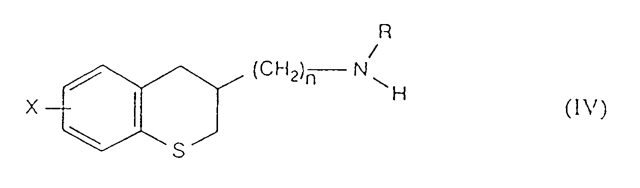 avec un agent alkylant R_{O}Y, où Y représente un groupe sortant, R un radical R₁ ou R₂ et R₀ l'autre radical R₂ ou R₁, ou bien R est un hydrogène et R₀Y est un agent alkylant bifonctionnel Y-R₁-R₂-Y, où R₁ et R₂ représentent ensemble un alkylène en C₄ à C₆ substitué par A, et où A, n, X, R₁ et R₂ ont les significations données ci-dessus, ou
(d) en réduisant un composé de formule 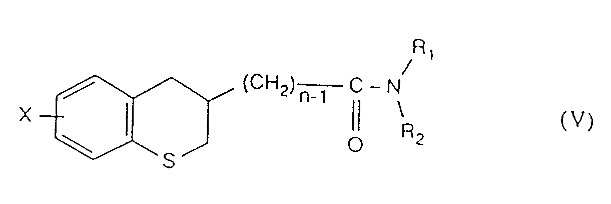 dans laquelle n, X, R₁ et R₂ ont les significations données ci-dessus, ou
(e) en réduisant un composé de formule 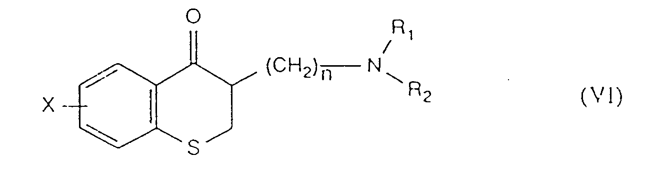 dans laquelle n, X, R₁ et R₂ ont les significations données ci-dessus,
et, si nécessaire en transformant un composé de formule I obtenu en un autre composé de formule I, où les substituants ont les significations données ci-dessus, et, si on le désire, en transformant un composé de formule I obtenu en un sel ou un sel d'un composé de formule I obtenu en le composé libre ou en un autre sel, et, si on le désire, en séparant un mélange d'isomères ou de racémates obtenu en les isomères ou racémates isolés et, si on le désire, en séparant un racémate obtenu en les antipodes optiques.

2. Procédé selon la revendication 1 de préparation de composés de formule I, où n vaut 1, X représente un hydrogène, halogène, alkyle inférieur ou aroyle inférieur, R₁ représente un alkyle inférieur, R₂ un alkyle inférieur ou un aroylalkyle inférieur, où R₁ et R₂ représentent ensemble un alkylène à chaîne droite en C₄ à C₆, qui est substitué par A et où A représente un hydrogène, hydroxyméthyle, α-hydroxyarylméthyle, α-hydroxydiarylméthyle, alcoxy inférieur méthyle, arylalcoxy inférieur méthyle, alcanoyloxy inférieur méthyle, arylalcanoyloxy inférieur méthyle, aroyloxyméthyle, alcanoyle inférieur, arylalcanoyle inférieur, aroyle, alcoxy inférieur carbonyle ou arylalcoxy inférieur carbonyle, et des sels de ces composés.

3. Procédé selon la revendication 1 de préparation de composés de formule I, dans laquelle n vaut 1 ou 2, X représente un hydrogène, halogène, alkyle inférieur ou alcoxy inférieur, R₁ est un alkyle inférieur, et R₂ est un alkyle inférieur ou un aroyle inférieur, ou R₁ et R₂ représentent ensemble un butylène ou pentylène à chaîne droite; et des sels d'addition d'acides pharmaceutiquement acceptables de ces composés.

4. Procédé selon la revendication 3 de préparation de composés de formule I, dans laquelle n vaut 1, X représente un hydrogène, halogène, alkyle inférieur ou alcoxy inférieur, R₁ est un alkyle inférieur et R₂ est un aroylalkyle inférieur; et des sels d'addition d'acides pharmaceutiquement acceptables de ces composés.

5. Procédé selon la revendication 3 de préparation de composés de formule I, dans laquelle n vaut 1, X représente un hydrogène, halogène, alkyle inférieur ou alcoxy inférieur et R₁ et R₂ représentent ensemble un butylène à chaîne droite; et des sels d'addition d'acides pharmaceutiquement acceptables de ces composés.

6. Procédé selon la revendication 3 de préparation de composés de formule I, dans laquelle n vaut 1, X représente un fluor et est en position 6, R₁ est un alkyle inférieur et R₂ est un p-fluorobenzoylalkyle inférieur, ou R₁ et R₂ représentent ensemble un butylène à chaîne droite; et des sels d'addition d'acides pharmaceutiquement acceptables de ces composés.

7. Procédé selon la revendication 1 de préparation de composés de formule I 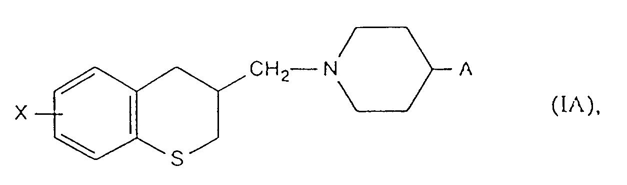 dans laquelle X représente un hydrogène, halogène, alkyle inférieur ou alcoxy inférieur et A représente un hydrogène, hydroxyméthyle, α-hydroxybenzyle, α-hydroxydiphénylméthyle, alcoxy inférieur méthyle, phénylalcoxy méthyle inférieur, alcanoyloxy inférieur méthyle, phénylalcanoyloxy inférieur méthyle, benzoylméthyle, alcanoyle inférieur, phénylalcanoyle inférieur, benzoyle, alcoxy inférieur carbonyle, ou phénylalcoxy inférieur carbonyle, et où le groupe phényle dans phényle, benzyle et benzoyle est non substitué ou substitué en position o, m ou p par un halogène; et des sels d'addition d'acides pharmaceutiquement acceptables de ces composés.

8. Procédé selon la revendication 7 de préparation de composés de formule IA, dans laquelle X représente un hydrogène, halogène, alkyle inférieur ou alcoxy inférieur, A représente un hydrogène, hydroxyméthyle, α-hydroxydi(p-fluorophényl)méthyle, alcoxy inférieur méthyle, p-fluorobenzyloxyméthyle, p-fluorobenzoyloxyméthyle, p-fluorobenzoyle ou alcoxy inférieur carbonyle; et des sels d'addition d'acides pharmaceutiquement acceptables de ces composés.

9. Procédé selon la revendication 7 de préparation de composés de formule IA, dans laquelle X représente un fluor et est en position 6 et A représente un α-hydroxydi-(p-fluorophényl)méthyle, p-fluorobenzyloxyméthyle, p-fluorobenzoyloxyméthyle ou p-fluorobenzoyle; et des sels d'addition d'acides pharmaceutiquement acceptables de ces composés.

10. Procédé selon la revendication 7 de préparation du composé de formule IA, dans laquelle X représente un fluor et est en position 6 et A représente un p-fluorobenzoyle; et des sels d'addition d'acides pharmaceutiquement acceptables de ce composé.

11. Procédé de préparation de compositions pharmaceutiques caractérisé en ce qu'on mélange un composé préparé selon la revendication 1 ou un de ses sels avec un support pharmaceutiquement acceptable.

12. Application d'un composé selon l'une des revendications 1 à 10 ou d'un de ses sels d'addition d'acides pharmaceutiquement acceptables à la préparation de préparations pharmaceutiques pour le traitement des désordres du système nerveux central, de l'appareil gastro-intestinal ou du système circulatoire cardiaque.
